(19) Europäisches Patentamt
European Patent Office
Office européen des brevets

(11) EP 1 893 200 B1

(12) **EUROPEAN PATENT SPECIFICATION**

(45) Date of publication and mention of the grant of the patent:
**29.09.2010 Bulletin 2010/39**

(21) Application number: **06744837.3**

(22) Date of filing: **17.05.2006**

(51) Int Cl.:
***A61K 31/445*** *(2006.01)* ***C07D 207/12*** *(2006.01)*
***C07D 211/42*** *(2006.01)* ***C07D 211/56*** *(2006.01)*
***C07D 401/12*** *(2006.01)* ***A61P 25/00*** *(2006.01)*

(86) International application number:
**PCT/IB2006/001612**

(87) International publication number:
**WO 2006/123244 (23.11.2006 Gazette 2006/47)**

(54) **CARBAMATE DERIVATIVES AS POSITIVE ALLOSTERIC MODULATORS OF METABOTROPIC GLUTAMATE RECEPTORS**

CARBAMAT-DERIVATE ALS POSITIVE ALLOSTERISCHE MODULATOREN VON METABOTROPISCHEN GLUTAMAT-REZEPTOREN

UTILISATION DE DERIVES DE CARBAMATE COMME MODULATEURS ALLOSTERIQUES POSITIFS DES RECEPTEURS METABOTROPIQUES DU GLUTAMATE

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.05.2005 GB 0510140**

(43) Date of publication of application:
**05.03.2008 Bulletin 2008/10**

(73) Proprietor: **ADDEX Pharma S.A.**
**1228 Plan-lès-Ouates (Geneva) (CH)**

(72) Inventors:
- **BUGADA, Piergiuliano**
  **I-20021 Baranzate (IT)**
- **GAGLIARDI, Stefania**
  **I-20021 Baranzate (IT)**
- **PALOMBI, Giovanni**
  **I-20021 Baranzate (IT)**
- **ROCHER, Jean-Philippe**
  **CH-1228 Plan les Ouates, Geneva (CH)**

(74) Representative: **Davies, Jonathan Mark et al**
**Reddie & Grose**
**16 Theobalds Road**
**London WC1X 8PL (GB)**

(56) References cited:
**WO-A-00/69816**
**WO-A-2004/014370**
**WO-A-2005/044797**
**WO-A1-2004/033440**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 893 200 B1

## Description

FIELD OF THE INVENTION

The present invention provides compounds of formula I-B and I-C as positive allosteric modulators of metabotropic receptors - subtype 5 ("mGluR5") which are useful for the treatment or prevention of central nervous system disorders such as for example: cognitive decline, both positive and negative symptoms in schizophrenia as well as other central or peripheral nervous system disorders in which the mGluR5 subtype of glutamate metabotropic receptor is involved. The invention is also directed to pharmaceutical compounds and compositions for prevention or treatment of such diseases in which mGluR5 is involved.

BACKGROUND OF THE INVENTION

Glutamate, the major amino-acid transmitter in the mammalian central nervous system (CNS), mediates excitatory synaptic neurotransmission through the activation of ionotropic glutamate receptors receptor-channels (iGluRs, namely NMDA, AMPA and kainate) and metabotropic glutamate receptors (mGluRs). iGluRs are responsible for fast excitatory transmission (Nakanishi S et al., (1998) Brain Res. Rev., 26:230-235) while mGluRs have a more modulatory role that contributes to the fine-tuning of synaptic efficacy. Glutamate performs numerous physiological functions such as long-term potentiation (LTP), a process believed to underlie learning and memory but also cardiovascular regulation, sensory perception, and the development of synaptic plasticity. In addition, glutamate plays an important role in the patho-physiology of different neurological and psychiatric diseases, especially when an imbalance in glutamatergic neurotransmission occurs.

The mGluRs are seven-transmembrane G protein-coupled receptors. The eight members of the family are classified into three groups (Groups I, II & III) according to their sequence homology and pharmacological properties (Schoepp DD et al. (1999) Neuropharmacology, 38:1431-1476). Activation of mGluRs lead to a large variety of intracellular responses and activation of different transductional cascades. Among mGluR members, the mGluR5 subtype is of high interest for counterbalancing the deficit or excesses of neurotransmission in neuropsychatric diseases. mGluR5 belongs to Group I and its activation initiates cellular responses through G-protein mediated mechanisms. mGluR5 is coupled to phospholipase C and stimulates phosphoinositide hydrolysis and intracellular calcium mobilization.

mGluR5 proteins have been demonstrated to be localized in post-synaptic elements adjacent to the post-synaptic density (Lujan R et al. (1996) Eur. J. Neurosci., 8:1488-500; Lujan R et al. (1997) J. Chem. Neuroanat., 13: 219-41) and are rarely detected in the pre-synaptic elements (Romano C et al. (1995) J. Comp. Neurol., 355:455-69). mGluR5 receptors can therefore modify the post-synaptic responses to neurotransmitter or regulate neurotransmitter release.

In the CNS, mGluR5 receptors are abundant mainly throughout the cortex, hippocampus, caudate-putamen and nucleus accumbens. As these brain areas have been shown to be involved in emotion, motivational processes and in numerous aspects of cognitive function, mGluR5 modulators are predicted to be of therapeutic interest.

A variety of potential clinical indications have been suggested to be targets for the development of subtype selective mGluR modulators. These include epilepsy, neuropathic and inflammatory pain, numerous psychiatric disorders (eg anxiety and schizophrenia), movement disorders (eg Parkinson disease), neuroprotection (stroke and head injury), migraine and addiction/drug dependency (for reviews, see Brauner-Osbome H et al. (2000) J. Med. Chem., 43:2609-45; Bordi F and Ugolini A. (1999) Prog. Neurobiol., 59:55-79; Spooren W et al. (2003) Behav. Pharmacol., 14:257-77).

The hypothesis of hypofunction of the glutamatergic system as reflected by NMDA receptor hypofunction as a putative cause of schizophrenia has received increasing support over the past few years (Goff DC and Coyle JT (2001) Am. J. Psychiatry, 158:1367-1377; Carlsson A et al. (2001) Annu. Rev. Pharmacol. Toxicol., 41:237-260 for a review). Evidence implicating dysfunction of glutamatergic neurotransmission is supported by the finding that antagonists of the NMDA subtype of glutamate receptor can reproduce the full range of symptoms as well as the physiologic manifestation of schizophrenia such as hypofrontality, impaired prepulse inhibition and enhanced subcortical dopamine release. In addition, clinical studies have suggested that mGluR5 allele frequency is associated with schizophrenia among certain cohorts (Devon RS et al. (2001) Mol. Psychiatry., 6:311-4) and that an increase in mGluR5 message has been found in cortical pyramidal cells layers of schizophrenic brain (Ohnuma T et al. (1998) Brain Res. Mol. Brain Res., 56:207-17).

The involvement of mGluR5 in neurological and psychiatric disorders is supported by evidence showing that in vivo activation of group I mGluRs induces a potentiation of NMDA receptor function in a variety of brain regions mainly through the activation of mGluR5 receptors (Mannaioni G et al. (2001) Neurosci., 21:5925-34; Awad H et al. (2000) J. Neurosci., 20:7871-7879; Pisani A et al. (2001) Neuroscience, 106:579-87; Benquet P et al (2002) J. Neurosci., 22: 9679-86).

The role of glutamate in memory processes also has been firmly established during the past decade (Martin SJ et al. (2000) Annu. Rev. Neurosci., 23:649-711; Baudry M and Lynch G. (2001) Neurobiol. Learn. Mem., 76:284-297).

The use of mGluR5 null mutant mice have strongly supported a role of mGluR5 in learning and memory. These mice show a selective loss in two tasks of spatial learning and memory, and reduced CA1 LTP (Lu et al. (1997) J. Neurosci., 17:5196-5205; Schulz B et al. (2001) Neuropharmacology, 41:1-7; Jia Z et al. (2001) Physiol. Behav., 73:793-802; Rodrigues et al. (2002) J. Neurosci., 22:5219-5229).

The finding that mGluR5 is responsible for the potentiation of NMDA receptor mediated currents raises the possibility that agonists of this receptor could be useful as cognitive-enhancing agents, but also as novel antipsychotic agents that act by selectively enhancing NMDA receptor function.

The activation of NMDARs could potentiate hypofunctional NMDARs in neuronal circuitry relevant to schizophrenia. Recent in vivo data strongly suggest that mGluR5 activation may be a novel and efficacious approach to treat cognitive decline and both positive and negative symptoms in schizophrenia (Kinney GG et al. (2003) J. Pharmacol. Exp. Ther., 306(1):116-123).

mGluR5 receptor is therefore being considered as a potential drug target for treatment of psychiatric and neurological disorders including treatable diseases in this connection are anxiety disorders, attentional disorders, eating disorders, mood disorders, psychotic disorders, cognitive disorders, personality disorders and substance-related disorders.

Most of the current modulators of mGluR5 function have been developed as structural analogues of glutamate, quisqualate or phenylglycine (Schoepp DD et al. (1999) Neuropharmacology, 38:1431-1476) and it has been very challenging to develop in vivo active and selective mGluR5 modulators acting at the glutamate binding site. A new avenue for developing selective modulators is to identify molecules that act through allosteric mechanisms, modulating the receptor by binding to site different from the highly conserved orthosteric binding site.

Positive allosteric modulators of mGluRs have emerged recently as novel pharmacological entities offering this attractive alternative. This type of molecule has been discovered for mGluRl, mGluR2, mGluR4, and mGluR5 (Knoflach F et al. (2001) Proc. Natl. Acad. Sci. U S A., 98:13402-13407; O'Brien JA et al. (2003) Mol. Pharmacol., 64:731-40; Johnson K et al. (2002) Neuropharmacology, 43:291; Johnson MP et al. (2003) J. Med. Chem., 46:3189-92; Marino MJ et al. (2003) Proc. Natl. Acad. Sci. U S A., 100(23):13668-73; for a review see Mutel V (2002) Expert Opin. Ther. Patents, 12:1-8; Kew JN (2004) Pharmacol. Ther., 104(3):233-44; Johnson MP et al. (2004) Biochem. Soc. Trans., 32:881-7). DFB and related molecules were described as in vitro mGluR5 positive allosteric modulators but with low potency (O'Brien JA et al. (2003) Mol. Pharmacol., 64:731-40). Benzamide derivatives have been patented (WO 2004/087048; O'Brien JA (2004) J. Pharmacol. Exp. Ther., 309:568-77) and aminopyrazole derivatives have been disclosed as mGluR5 positive allosteric modulators (Lindsley et al. (2004) J. Med. Chem., 47:5825-8; WO 2005/087048). Among aminopyrazole derivatives, CDPPB has shown in vivo activity antipsychotic-like effects in rat behavioral models (Kinney GG et al. (2005) J. Pharmacol. Exp. Ther., 313:199-206). This report is consistent with the hypothesis that allosteric potentiation of mGluR5 may provide a novel approach for development of antipsychotic agents. Recently a novel series of positive allosteric modulators of mGluR5 receptors has been disclosed (WO 2005/044797).

None of the specifically disclosed compounds are structurally related to the compounds of the present invention.

The present invention relates to compounds of formula I-B and of formula I-C, as in claim 1, wherein Q is phenyl optionally substituted as indicated in claim 1 and wherein J is a bond or a $CH_2$ group.

The present compounds consist of a piperidine ring bound in position 3 to a cyclic group P through a linker which is either a -N(CO)O- or a -N(CO)N- moiety.

The technical problem underlying the present invention is the provision of glutamate receptor modulators (mGluR5 modulators).

WO 2004/014370 which relates to mGluR5 modulators represents prior art but does not teach nor suggest the use as mGluR5 modulators of derivatives having a piperidine ring bound in position 3 to a ring P through a linker which is either a -N(CO)O- or a -N(CO)N- moiety.

WO 2004/033440 discloses compounds falling within the scope of formula I-C of present claim 1. These compounds are intermediates (no activity disclosed for said compounds) and hence accidential disclosures. They are disclaimed by way of proviso in present claim 1.

FIGURE

Figure 1 shows the effect of 10 μM of example #1 of the present invention on primary cortical mGluR5-expressing cell cultures in the absence or in the presence of 300nM glutamate.

DETAILED DESCRIPTION OF THE INVENTION

According to the present invention, there are provided compounds of the general formula I-B and I-C, as defined

in the attached claims.

The present invention includes both possible stereoisomers and includes not only racemic compounds but the individual enantiomers as well.

For the avoidance of doubt it is to be understood that in this specification "($C_1$-$C_6$)" means a carbon group having 1, 2, 3, 4, 5 or 6 carbon atoms. "($C_0$-$C_6$)" means a carbon group having 0, 1, 2, 3, 4, 5 or 6 carbon atoms.

In this specification "C" means a carbon atom.

In the above definition, the term "($C_1$-$C_6$)alkyl" includes group such as methyl, ethyl, propyl, isopropyl, butyl, isobutyl, sec-butyl, tert-butyl, pentyl, isopentyl, neopentyl, tert-pentyl, hexyl or the like.

"($C_2$-$C_6$)alkenyl" includes group such as ethenyl, 1-propenyl, allyl, isopropenyl, 1-butenyl, 3-butenyl, 4-pentenyl and the like.

"($C_2$-$C_6$)alkynyl" includes group such as ethynyl, propynyl, butynyl, pentynyl and the like.

"Halogen" includes atoms such as fluorine, chlorine, bromine and iodine.

"Cycloalkyl" refers to an optionally substituted carbocycle containing no heteroatoms, includes mono-, bi-, and tricyclic saturated carbocycles, as well as fused ring systems. Such fused ring systems can include on ring that is partially or fully unsaturated such as a benzene ring to form fused ring systems such as benzo fused carbocycles. Cycloalkyl includes such fused ring systems as spirofused ring systems. Examples of cycloalkyl include cyclopropyl, cyclobutyl, cyclopentyl, cyclohexyl, decahydronaphthalene, adamantane, indanyl, fluorenyl, 1,2,3,4-tetrahydronaphthalene and the like.

"Heterocycloalkyl" refers to an optionally substituted carbocycle containing at least one heteroatom selected independently from O, N, S. It includes mono-, bi-, and tricyclic saturated carbocycles, as well as fused ring systems. Such fused ring systems can include one ring that is partially or fully unsaturated such as a benzene ring to form fused ring systems such as benzo fused carbocycles. Examples of heterocycloalkyl include piperidine, piperazine, morpholine, tetrahydrothiophene, indoline, isoquinoline and the like.

"Aryl" includes ($C_6$-$C_{10}$)aryl group such as phenyl, 1-naphtyl, 2-naphtyl and the like.

"Arylalkyl" includes ($C_6$-$C_{10}$)aryl-($C_1$-$C_3$)alkyl group such as benzyl group, 1-phenylethyl group, 2-phenylethyl group, 1-phenylpropyl group, 2-phenylpropyl group, 3-phenylpropyl group, 1-naphtylmethyl group, 2-naphtylmethyl group or the like.

"Heteroaryl" includes 5-10 membered heterocyclic group containing 1 to 4 heteroatoms selected from oxygen, nitrogen or sulphur to form a ring such as furyl (furan ring), benzofuranyl (benzofuran ring), thienyl (thiophene ring), benzothiophenyl (benzothiophene ring), pyrrolyl (pyrrole ring), imidazolyl (imidazole ring), pyrazolyl (pyrazole ring), thiazolyl (thiazole ring), isothiazolyl (isothiazole ring), triazolyl (triazole ring), tetrazolyl (tetrazole ring), pyridil (pyridine ring), pyrazynyl (pyrazine ring), pyrimidinyl (pyrimidine ring), pyridazinyl (pyridazine ring), indolyl (indole ring), isoindolyl (isoindole ring), benzoimidazolyl (benzimidazole ring), purinyl group (purine ring), quinolyl (quinoline ring), phtalazinyl (phtalazine ring), naphtyridinyl (naphtyridine ring), quinoxalinyl (quinoxaline ring), cinnolyl (cinnoline ring), pteridinyl (pteridine ring), oxazolyl (oxazole ring), isoxazolyl (isoxazole ring), benzoxazolyl (benzoxazole ring), benzothiazolyly (benzothiaziole ring), furazanyl (furazan ring) and the like.

"Heteroarylalkyl" includes heteroaryl-($C_1$-$C_3$-alkyl) group, wherein examples of heteroaryl are the same as those illustrated in the above definition, such as 2-furylmethyl group, 3-furylmethyl group, 2-thienylmethyl group, 3-thienylmethyl group, 1-imidazolylmethyl group, 2-imidazolylmethyl group, 2-thiazolylmethyl group, 2-pyridylmethyl group, 3-pyridylmethyl group, 1-quinolylmethyl group or the like.

"Solvate" refers to a complex of variable stoechiometry formed by a solute (e.g. a compound of formula I) and a solvent. The solvent is a pharmaceutically acceptable solvent as water preferably; such solvent may not interfere with the biological activity of the solute.

"Optionally" means that the subsequently described event(s) may or may not occur, and includes both event (s), which occur, and events that do not occur.

The term "substituted" refers to substitution with the named substituent or substituents, multiple degrees of substitution being allowed unless otherwise stated.

The term "general formula I" refers to formula I-B and/or I-C.

Specifically preferred compounds are:

1-[(S)-1-(4-Fluoro-benzoyl)-piperidin-3-yl]-3-(4-fluoro-phenyl)-urea (4-Fluoro-phenyl)-carbamic acid (S)-1-(4-fluoro-benzoyl)-piperidin-3-yl ester (4-Chloro-phenyl)-carbamic acid (S)-1-(4-fluoro-benzoyl)-piperidin-3-yl ester (4-Methoxy-phenyl)-carbamic acid (S)-1-(4-fluoro-benzoyl)-piperidin-3-yl ester Phenyl-carbamic acid (S)-1-(4-fluoro-benzoyl)-piperidin-3-yl ester (3-Fluoro-phenyl)-carbamic acid (S)-1-(4-fluoro-benzoyl)-piperidin-3-yl ester (2-Fluoro-phenyl)-carbamic acid (S)-1-(4-fluoro-benzoyl)-piperidin-3-yl ester Cyclopentyl-carbamic acid (S)-1-(4-fluoro-benzoyl)-piperidin-3-yl ester Cyclohexyl-carbamic acid (S)-1-(4-fluoro-benzoyl)-piperidin-3-yl ester Pyridin-4-yl-carbamic acid (S)-1-(4-fluoro-benzoyl)-piperidin-3-yl ester (4-Fluoro-benzyl)-carbamic acid (S)-1-(4-fluoro-benzoyl)-piperidin-3-yl ester (Phenyl)-carbamic acid-1-(4-fluoro-benzoyl)-pyrrolidin-3-yl ester

Compounds and compositions of the present invention may be used for treating or preventing a condition in a

mammal, including a human, the treatment or prevention of which is affected or facilitated by the neuromodulatory effect of mGluR5 allosteric modulators and particularly positive allosteric modulators.

Compounds and compositions of the present invention may be useful for treating or preventing various peripheral and central nervous system disorders such as tolerance or dependence, anxiety, depression, psychiatric disease such as psychosis, inflammatory or neuropathic pain, memory impairment, Alzheimer's disease, ischemia, drug abuse and addiction, as defined in the attached claims.

Pharmaceutical compositions of the invention may provide from about 0.01 to 1000 mg of the active ingredient per unit dose. The compositions may be administered by any suitable route. For example orally in the form of capsules, parenterally in the form of solutions for injection, topically in the form of ointments or lotions, ocularly in the form of eye-lotion, rectally in the form of suppositories.

The pharmaceutical formulations of the invention may be prepared by conventional methods in the art; the nature of the pharmaceutical composition employed will depend on the desired route of administration. The total daily dose usually ranges from about 0.05 - 2000 mg.

METHODS OF SYNTHESIS

Compounds of general formula I may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (Green T.W. and Wuts P.G.M. (1991) Protecting Groups in Organic Synthesis, John Wiley et Sons ). These groups are removed at a convenient stage of the compound synthesis using methods that are readily apparent to those skilled in the art. The selection of process as well as the reaction conditions and order of their execution shall be consistent with the preparation of compounds of formula I. The compound of formula I may be represented as a mixture of enantiomers, which may be resolved into the individual pure R- or S-enantiomers. If for instance, a particular enantiomer of the compound of formula I is desired, it may be prepared by asymmetric synthesis, or by derivation with a chiral auxiliary, where the resulting diastereomeric mixture is separated and the auxiliary group cleaved to provided the pure desired enantiomers. Alternatively, where the molecule contains a basic functional group such as amino, or an acidic functional group such as carboxyl, this resolution may be conveniently performed by fractional crystallization from various solvents, of the salts of the compounds of formula I with optical active acid or by other methods known in the literature, e.g. chiral column chromatography. Resolution of the final product, an intermediate or a starting material may be performed by any suitable method known in the art as described by Eliel E.L., Wilen S.H. and Mander L.N. (1984) Stereochemishy of Organic Compounds, Wiley-Interscience.

Many of the heterocyclic compounds of formula I where A is an heteroaromatic group can be prepared using synthetic routes well known in the art (Katrizky A.R. and. Rees C.W. (1984) Comprehensive Heterocyclic Chemistry, Pergamon Press). The product from the reaction can be isolated and purified employing standard techniques, such as extraction, chromatography, crystallization, distillation, and the like.

The compounds of formula **I-B** wherein J is $CH_2$ may be prepared according to the synthetic sequences illustrated in the following Schemes.

Wherein

P and Q each independently is aryl or heteroaryl as described above

B represents -C(=O)-$C_0$-$C_2$-alkyl-.

Compound **I-B** can be prepared by methods known in the art of organic synthesis as set forth in part by the following synthesis Scheme 1.

Scheme 1

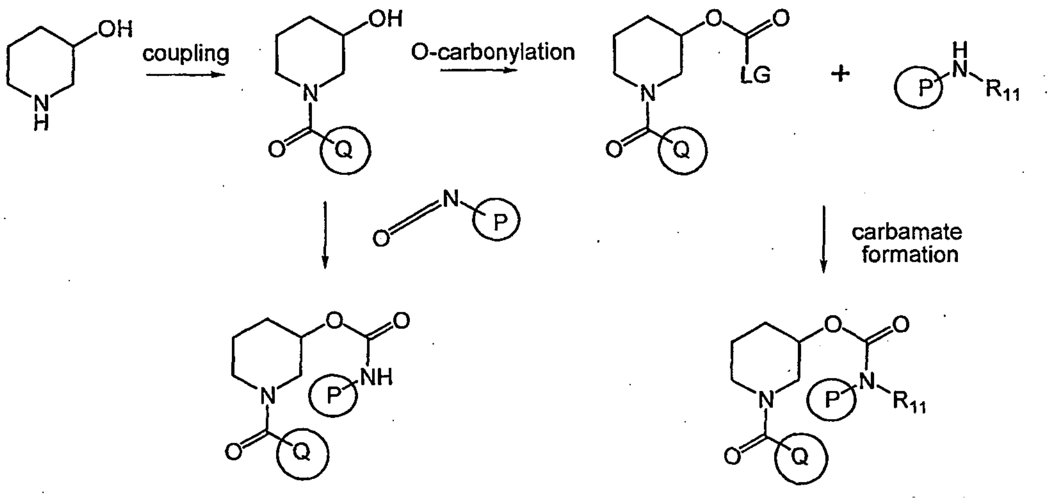


The coupling reaction may be promoted by coupling agents known in the art of organic synthesis such as EDCI (1-(3-dimethylaminopropyl)-3-ethylcarbodiimide), DCC (N,N'-dicyclohexyl-carbodiimide), in the presence of a suitable base such as triethylamine, diisopropyl-ethylamine, in a suitable solvent (e.g. tetrahydrofuran, dichloromethane, N,N-dimethylformamide, dioxane). Typically, a co-catalyst such as HOBT (hydroxy-benzotriazole), HOAT (1-hydroxy-7-azabenzotriazole) may also be present in the reaction mixture. The reaction typically proceeds at a temperature in the range of ambient temperature up to 60°C inclusive for a time in the range of about 2 hours up to 12 hours to produce the intermediate acyl-piperidinol.

Alternatively, the coupling reaction may be performed via acyl chloride, that is condensation with an aryl or heteroaryl acyl chloride using methods that are readily apparent to those skilled in the art. The reaction may be promoted by a base such as triethylamine, diisopropylamine, pyridine in a suitable solvent (*e.g.* tetrahydrofuran, dichloromethane). The reaction typically proceeds by allowing the reaction temperature to warm slowly from 0°C up to ambient temperature for a time in the range of about 4 up to 12 hours.

The O-carbonylation reaction may be promoted by reaction of the hydroxy derivative with for example: hexachloroacetone, trichloromethylchloroformate, triphosgene (carbonic acid ditrichloromethyl ester) in the presence of a suitable base such as triethylamine, diisopropyl-ethylamine, in a suitable solvent (e.g. tetrahydrofuran, dichloromethane). The reaction typically proceeds at a temperature in the range of ambient temperature up to 60°C inclusive for a time in the range of about 1 hour up to 4 hours to produce the intermediate acyloxy-piperidine. In the final step the displacement of the leaving group (LG in Scheme 1) may be effected using an amine in the presence of a suitable base such as triethylamine, diisopropyl-ethylamine, in a suitable solvent (e.g. tetrahydrofuran, dichloromethane, N,N-dimethylformamide, dioxane). The reaction typically proceeds at a temperature in the range of ambient temperature up to 40°C inclusive for a time in the range of about 1 hour up to 5 hours to produce compound **I-B** (see for example Tetrahedron Lett., 1992, 3583-3586; Bioorg. Med Chem, 2003, 4315- J. Org. Chem. 1981, 46, 3519-3521; Tetrahedron, 1998, 6757-).

When $R_{11}$ = H, the carbamoylation can be performed by reacting the hydroxyl intermediate with an isocyanate under classical conditions ( Smith M.B.and March J. 2001, March's advanced Organic Chemistry, John Wiley &Sons).

The compounds of formula **I-C** wherein J is $CH_2$ may be prepared according to the synthetic sequences illustrated in Scheme 2.

Wherein

P and Q each independently is aryl or heteroaryl as described above

B represents -C(=O)-$C_0$-$C_2$-alkyl-; -S(=O)2-$C_0$-$C_2$-alkyl-.

The urea described below is prepared following synthetic routes well know in the art (French, Wirtel, Am. Chem. J, 1926, 48, 1736).

Scheme 2

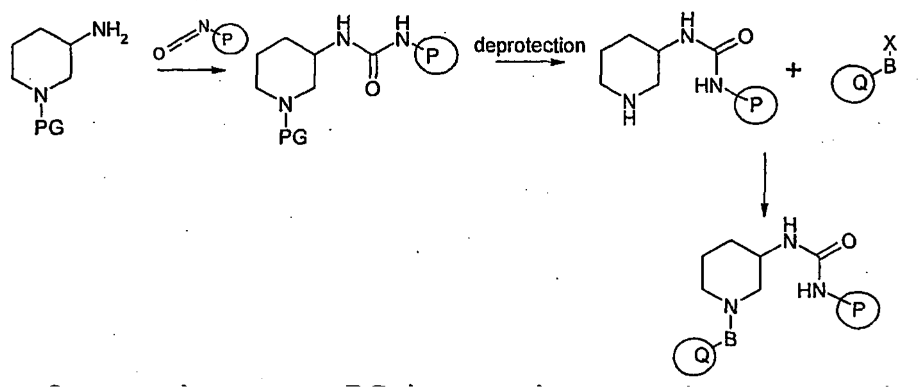


In Scheme 2, protecting groups PG is an amino protecting group such as *tert*-butyloxycarbonyl, benzyloxycarbonyl, ethoxycarbonyl, benzyl and the like. The urea formation may be promoted using isocyanate derivatives in a suitable solvent (such as tetrahydrofuran, dichloromethane). The reaction typically proceeds at room temperature for a time in the range of about 1 hour up to 5 hours. As shown in the Scheme 2, protecting groups $PG_1$ are removed using standard methods.

The final step may be effected either by a process described in the Scheme 2 or by a process described in the Scheme 3. As shown in the Scheme 2 B is as defined above, X is halogen, for example the piperidine derivative is reacted with an aryl or heteroaryl acyl chloride using method that are readily apparent to those skilled in the art. The reaction may be promoted by a base such as triethylamine, diisopropylamine, pyridine in a suitable solvent (*e.g.* tetrahydrofuran, dichloromethane). The reaction typically proceeds by allowing the reaction temperature to warm slowly from 0°C up to ambient temperature for a time in the range of about 4 up to 12 hours.

Scheme 3

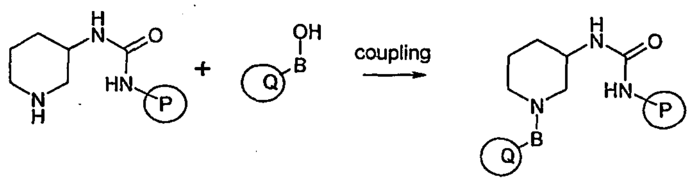


As shown in the Scheme 3, The coupling reaction may be promoted by coupling agents known in the art of organic synthesis such as EDCI (1-(3-dimethylaminopropyl)-3-ethylcarbodiimide), DCC (N,N'-dicyclohexyl-carbodiimide) or by polymer-supported coupling agents such as polymer-supported carbodiimide (PS-DCC, ex Argonaut Technologies), in the presence of a suitable base such as triethylamine, diisopropyl-ethylamine, in a suitable solvent (e.g. tetrahydrofuran, dichloromethane, N,N-dimethylformamide, dioxane). Typically, a co-catalyst such as HOBT (1-hydroxybenzotriazole), HOAT (1-hydroxy-7-azabenzotriazole) and the like may also be present in the reaction mixture. The reaction typically proceeds at ambient temperature for a time in the range of about 2 hours up to 12 hours.

The compounds of Formula **I** which are basic in nature can form a wide variety of different pharmaceutically acceptable salts with various inorganic and organic acids. These salts are readily prepared by treating the base compounds with a substantially equivalent amount of the chosen mineral or organic acid in a suitable organic solvent such as methanol, ethanol or isopropanol (see Stahl P.H., Wermuth C.G., Handbook of Pharmaceuticals Salts, Properties, Selection and Use, Wiley, 2002).

The following non-limiting examples are intending to illustrate the invention. The physical data given for the compounds exemplified is consistent with the assigned structure of those compounds.

EXAMPLES

Unless otherwise noted, all starting materials were obtained from commercial suppliers and used without further purification.

Specifically, the following abbreviation may be used in the examples and throughout the specification.

| | |
|---|---|
| g (grams) | RT (retention time) |
| mg (milligrams) | MeOH (methanol) |
| mL (millilitres) | |
| υl (microliters) | Hz (Hertz) |
| M (molar) | LCMS (Liquid Chromatography Mass Spectrum) |
| MHz (megahertz) | HPLC (High Pressure Liquid Chromatography) |
| mmol (millimoles) | NMR (Nuclear Magnetic Resonance) |
| min (minutes) | 1H (proton) |
| AcOEt (ethyl acetate) | $Na_2SO_4$ (sodium sulphate) |
| $K_2CO_3$ (potassium carbonate) | $MgSO_4$ (magnesium sulphate) |
| $CDCl_3$ (deuterated chloroform) | HOBT (1-hydroxybenzotriazole) |
| EDC.HCl (1-3(Dimethylaminopropyl)-3-ethylcarbodiimide, hydrochloride) | r.t. (Room Temperature) |
| EtOH (ethyl alcohol) | NaOH (sodium hydroxide) |
| % (percent) | h (hour) |
| DCM (dichloromethane) | HCl (hydrochloric acid) |
| DIEA (diisopropyl ethyl amine) | n-BuLi (n-butyllithium) |
| Mp (melting point) | THF (tetrahydrofuran) |

All references to brine refers to a saturated aqueous solution of NaCl. Unless otherwise indicated, all temperatures are expressed in °C (degrees Centigrade). All reactions are conducted under an inert atmosphere at room temperature unless otherwise noted.
$^1$H NMR spectra were recorded on a Brucker 300MHz. Chemical shifts are expressed in parts of million (ppm, δ units). Coupling constants are in units of hertz (Hz) Splitting patterns describe apparent multiplicities and are designated as s (singlet), d (doublet), t (triplet), q (quadruplet), quint (quintuplet), m (multiplet).

LCMS were recorded under the following conditions:

Method A) Waters Alliance 2795 HT Micromass ZQ. Column Waters XTerra MS C18 (50x4.6 mm, 2.5μm). Flow rate 1 ml/min Mobile phase: A phase = water/$CH_3CN$ 95/5 + 0.05% TFA, B phase = water/$CH_3CN$ = 5/95 + 0.05% TFA. 0-1 min (A: 95%, B: 5%), 1-4 min (A: 0%, B: 100%), 4-6 min (A: 0%, B: 100%), 6-6.1 min (A: 95%, B: 5%). T= 35°C; UV detection: Waters Photodiode array 996, 200-400nm.

Method B) Waters Alliance 2795 HT Micromass ZQ. Column Waters Symmetry C18 (75x4.6 mm, 3.5μm). Flow rate 1 ml/min Mobile phase: A phase = water/$CH_3CN$ 95/5 + 0.05% TFA, B phase = water/$CH_3CN$ = 5/95 + 0.05% TFA. 0-0.1 min (A: 95%, B: 5%), 1-11 min (A: 0%, B: 100%), 11-12 min (A: 0%, B: 100%), 12-12.1 min (A: 95%, B: 5%). T= 35°C; UV detection: Waters Photodiode array 996, 200-400nm.

Method C): Pump 515, 2777 Sample Manager, Micromass ZQ Single quadrupole (Waters). Column 2.1*50mm stainless steel packed with 3.5μm SunFire RP C-18 (Waters); flow rate 0.25 ml/min splitting ratio MS :waste/ 1:4; mobile phase: A phase = water/acetonitrile 95/5 + 0.1% TFA, B phase = water/acetonitrile 5/95 + 0.1% TFA. 0-1.0min (A: 98%, B: 2%), 1.0-5.0min (A: 0%, B: 100%), 5.0-9.0min (A: 0%, B: 100%), 9.1-12min (A: 98%, B: 2%); UV detection wavelength 254 nm; Injection volume: 5μl

Method D): Pump 1525u (Waters), 2777 Sample Manager, Micromass ZQ2000 Single quadrupole (Waters); PDA detector: 2996 (Waters). Column 2.1 *30mm stainless steel packed with 3.0μm Luna C18; flow rate 0.25 ml/min splitting ratio MS :waste/ 1:4; mobile phase: A phase = water/acetonitrile 95/5 + 0.1% TFA, B phase = water/acetonitrile 5/95 + 0.1 % TFA. 0-1.0min (A: 98%, B: 2%), 1.0-5.0min (A: 0%, B: 100%), 5.0-9.0min (A: 0%, B: 100%), 9.1-12min (A: 98%, B: 2%); UV detection wavelenght 254 nm; Injection volume: 5μl.

Method E): LCMS were recorded on a Waters Micromass ZQ 2996 system by the following conditions: Column 4.6*30mm stainless steel packed with 1.8μm Zorbax SB C-18; flow rate 1.5ml/min; mobile phase: A phase = 0.05% formic acid in water, B phase = 0.05% formic acid in acetonitrile. 0-3.5min (A: 90%, B: 10%), 3.5-3.7min (A: 0%, B: 100%), 3.8-4.5min (A: 90%, B: 10%). Oven temperature: 30°C ±1°C; UV detection Diode Array: 200-400nm.

All mass spectra were taken under electrospray ionisation (ESI) methods.

The microwave oven used is an apparatus from Biotage (Optimizer™) equipped with an internal probe that monitors reaction temperature and pressure, and maintains the desired temperature by computer control.

Most of the reactions were monitored by thin-layer chromatography on 0.25mm Macherey-Nagel silica gel plates (60F-2254), visualized with UV light. Flash column chromatography was performed on silica gel (220-440 mesh, Fluka).

Melting point determination was performed on a Buchi B-540 apparatus.

Example 1

(4-Fluoro-phenyl)-carbamic acid (S)-1-(4-fluoro-benzoyl)-piperidin-3-yl ester

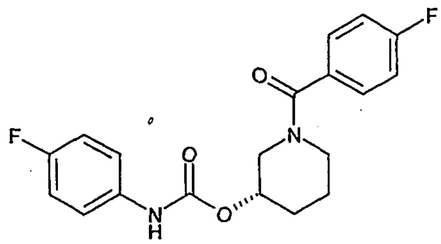

1(A) (4-Fluoro-phenyl)-((S)-3-hydroxy-piperidin-1-yl)-methanone

A mixture of (S)-3-hydroxy piperidine hydrochloride (0.2 g, 1.45 mmol), 4-fluoro benzoic acid (0.204 g, 1.45 mmol), EDC.HCl (0.42 g, 2.18 mmol), HOBT (0.196 g, 1.45 mmol), triethylamine (320 uL, 4.36 mmol) in dichloromethane (10 mL) was stirred under nitrogen atmosphere overnight at room temperature. The reaction mixture was diluted with dichloromethane (20 mL) and washed subsequently with 0.1 N HCl (2 times), 0.1 N NaOH (2 times) and then with brine. The organic layer was dried over sodium sulphate and evaporated under reduced pressure to give a pale yellow oil (275 mg), which was used for the next step without further purification.
Yield: 84%; LCMS (RT): 2.5 min (Method A); MS (ES+) gave m/z: 224.1.

1(B) (4-Fluoro-phenyl)-((S)-3-(chloroformate)-piperidin-1-yl)-methanone

Triethylamine (375 uL, 2.7 mmol) and then triphosgene (400 mg, 1.35 mmol) were dropped into a solution of (4-fluoro-phenyl)-((S)-3-hydroxy-piperidin-1-yl)-methanone (200 mg, 0.9 mmol) in DCM (5 mL) at room temperature, under nitrogen atmosphere. After stirring 1h, water and ethyl acetate were added (25 mL) and the phases were separated. The organic layer was dried over sodium sulphate and evaporated under reduced pressure to provide a crude oil (331 mg), which was used for the next step without further purification.
Yield: 100%; LCMS (RT): 4.7 min (Method A); MS (ES+) gave m/z: 345, 285.9 and 287.9.

1(C) (4-Fluoro-phenyl)-carbamic acid (S)-1-(4-fluoro-benzoyl)-piperidin-3-yl ester

Triethylamine (250 uL, 1.8 mmol) and then 4-fluoroaniline (102 uL, 1.1 mmol) were dropped into a solution of (4-Fluoro-phenyl)-((S)-3-(chloroformate)-piperidin-1-yl)-methanone (331 mg, 0.9 mmol) in DCM (5 mL) at room temperature, under nitrogen atmosphere. After stirring 2h, 10% HCl was added and the phases were separated, the organic layer was dried over magnesium sulphate and evaporated to dryness under reduced pressure. The crude residue was purified by flash chromatography (silica gel, eluent: petroleum ether/ethyl acetate 6:4) and then by preparative HPLC to afford the title compound (30 mg) as a gummy yellow solid.
Yield: 9%; LCMS (RT): 6.93 min (Method C); MS (ES+) gave m/z: 361.2.
$^1$H-NMR (DMSO-$d_6$, 300 MHz), δ (ppm): 9.11 (s br, 1H); 7.48-7.39(m, 4H); 7.13(dd, 2H); 7.06(dd, 2H); 4.75(m, 1H);

3.75-3.53(m, 3H); 3.34(m, 1H); 1.98(m; 1H); 1.89-1.74(m, 2H); 1.59(m, 1H).

Example 2

(4-Chloro-phenyl)-carbamic acid (S)-1-(4-fluoro-benzoyl)-piperidin-3-yl ester

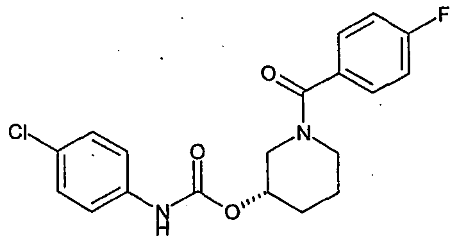

To a solution of (4-fluoro-phenyl)-((S)-3-hydroxy-piperidin-1-yl)-methanone (100 mg, 0.45 mmol), prepared as described in Example 1(A), and triethylamine (63 uL, 0.45 mmol) in dichloromethane, 4-chlorophenyl isocyanate (138 mg, 0.90 mmol) was added at room temperature, under nitrogen atmosphere. After stirring at RT for 6h, the solvent was evaporated under reduced pressure and the resulting crude residue was purified by flash chromatography (silica gel, eluent: petroleum ether / ethyl acetate 1:1). The solid compound obtained after column chromatography was repurified by preparative HPLC to afford (4-chloro-phenyl)-carbamic acid (S)-1-(4-fluorobenzoyl)-piperidin-3-yl ester (53 mg).
Yield: 31% (colourless oil); $[\alpha]_D^{20}$ = +2.21° (c=0.33, MeOH); LCMS (RT): 6.65 min (Method D); MS (ES+) gave m/z: 377.2.
$^1$H-NMR (DMSO-$d_6$, 343K), δ (ppm): 9.48 (s br, 1H); 7.44 (m, 4H); 7.30 (d, 1H); 7.13 (dd, 2H); 4.75 (m, 1H); 3.74-3.54 (m, 3H); 3.34 (m, 2H); 1.95 (m, 1H); 1.88-1.78 (m, 2H); 1.59 (m, 1H).

Example 3

(4-Methoxy-phenyl)-carbamic acid (S)-1-(4-fluoro-benzoyl)-piperidin-3-yl ester

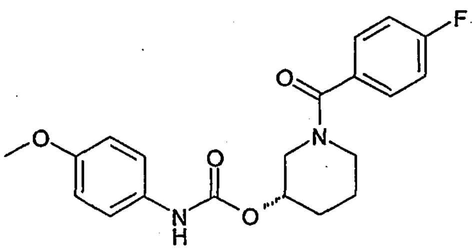

To a solution of (4-fluoro-phenyl)-((S)-3-hydroxy-piperidin-1-yl)-methanone (100 mg, 0.45 mmol), prepared as described in Example 1(A), in dichloromethane (2 mL), 4-methoxyphenyl isocyanate (90 uL, 0.675 mmol) was added at room temperature, under nitrogen atmosphere. After stirring at RT for 6h, the solvent was evaporated under reduced pressure and the resulting crude residue was purified by preparative HPLC to afford (4-methoxy-phenyl)-carbamic acid (S)-1-(4-fluoro-benzoyl)-piperidin-3-yl ester (110 mg).
Yield: 66% (black oil); $[\alpha]_D^{20}$ = +6.03° (c=1.0, MeOH); LCMS (RT): 6.35 min (Method D); MS (ES+) gave m/z: 373.1.
$^1$H-NMR (DMSO-$d_6$, 373K +TFA), δ (ppm): 9.05 (s br, 1H); 7.44 (dd, 2H); 7.32 (d, 2H); 7.14 (dd, 2H); 6.85 (d, 2H); 4.73 (m, 1H); 3.74 (s, 3H); 3.68 (d br, 2H); 3.56 (dd, 1H); 3.33 (m, 1H); 1.96 (m, 1H); 1.88-1.73 (m, 2H); 1.58 (m, 1H).

Example 4

Phenyl-carbamic acid (S)-1-(4-fluoro-benzoyl)-piperidin-3-yl ester

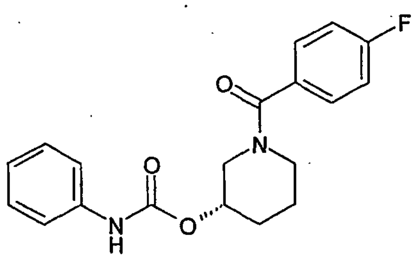

To a solution of (4-fluoro-phenyl)-((S)-3-hydroxy-piperidin-1-yl)-methanone (100 mg, 0.45 mmol), prepared as described in Example 1(A), in dichloromethane (1 mL), phenyl isocyanate (98 uL, 0.90 mmol) was added at room temperature, under nitrogen atmosphere. After stirring at 50°C for 6h, the solvent was evaporated under reduced pressure and the resulting crude residue was purified by flash chromatography (silica gel, eluent gradient: from petroleum ether / ethyl acetate 7:3 to petroleum ether / ethyl acetate 1:1). The solid compound obtained after column chromatography was repurified by preparative HPLC to afford phenyl-carbamic acid (S)-1-(4-fluorobenzoyl)-piperidin-3-yl ester (46 mg).
Yield: 30% (white solid); $[\alpha]_D^{20}$ = +2.91° (c=1.0, MeOH); LCMS (RT): 6.46 min (Method D); MS (ES+) gave m/z: 343.3.
$^1$H-NMR (DMSO-$d_6$, 373K), δ (ppm): 8.99 (s br, 1H); 7.44 (m, 4H); 7.26 (dd, 2H); 7.12 (dd, 2H); 7.01 (dd, 1H); 4.77 (m, 1H); 3.71 (dd, 1H); 3.64 (m, 1H); 3.58 (dd, 1H); 3.37 (ddd, 1H); 1.99 (m, 1H); 1.91-1.75 (m, 2H); 1.60 (m, 1H).

Example 5

(3-Fluoro-phenyl)-carbamic acid (S)-1-(4-fluoro-benzoyl)-piperidin-3-yl ester

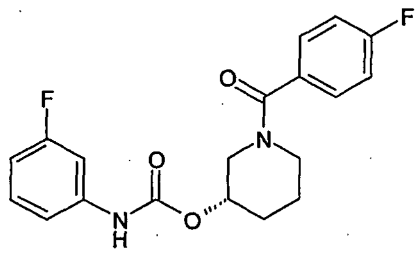

The title compound was obtained following the experimental procedure described in Example 4, starting from (4-fluoro-phenyl)-((S)-3-hydroxy-piperidin-1-yl)-methanone, prepared as described in Example 1(A), and 3-fluorophenyl isocyanate. (3-Fluoro-phenyl)-carbamic acid (S)-1-(4-fluoro-benzoyl)-piperidin-3-yl ester was obtained pure after purification by flash chromatography (silica gel, eluent gradient: from petroleum ether / ethyl acetate 7:3 to petroleum ether / ethyl acetate 1:1).
Yield: 63% (white solid); $[\alpha]_D^{20}$ = +5.03° (c=0.9, MeOH); LCMS (RT): 6.57 min (Method D); MS (ES+) gave m/z: 361.2.
$^1$H-NMR (DMSO-$d_6$, 373K), δ (ppm): 9.29 (s br, 1H); 7.44 (dd, 2H); 7.33-7.20 (m, 3H); 7.13 (dd, 2H); 6.78 (m, 1H); 4.78 (m, 1H); 3.71 (dd, 1H); 3.65 (m, 1H); 3.60 (dd, 1H); 3.36 (ddd, 1H); 1.99 (m, 1H); 1.90-1.76 (m, 2H); 1.59 (m, 1H).

Example 6

(2-Fluoro-phenyl)-carbamic acid (S)-1-(4-fluoro-benzoyl)-piperidin-3-yl ester

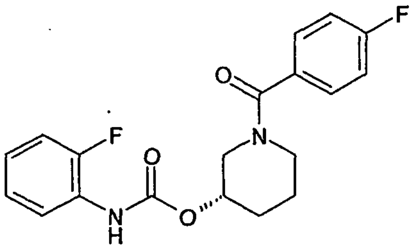

The title compound was obtained following the experimental procedure described in Example 4, starting from (4-fluoro-phenyl)-((S)-3-hydroxy-piperidin-1-yl)-methanone, prepared as described in Example 1(A), and 2-fluorophenyl isocyanate.
Yield: 38% (colourless oil); $[\alpha]_D^{20}$ = +4.21° (c=0.98, MeOH); LCMS (RT): 6.42 min (Method D); MS (ES+) gave m/z: 361.2. $^1$H-NMR (DMSO-$d_6$, 373K), δ (ppm): 8.62 (s br, 1H); 7.56 (m, 1H); 7.42 (dd, 2H); 7.20-7.09 (m, 5H); 4.76 (m, 1H); 3.68 (dd, 1H); 3.64 (m, 1H); 3.59 (dd, 1H); 3.34 (ddd, 1H); 1.96 (m, 1H); 1.88-1.74 (m, 2H); 1.58 (m, 1H).

Example 7

Cyclopentyl-carbamic acid (S)-1-(4-fluoro-benzoyl)-piperidin-3-yl ester

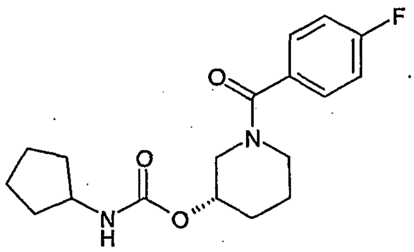

A solution of cyclopentylamine (165 uL, 1.67 mmol) and (S)-carbonic acid 1-(4-fluoro-benzoyl)-piperidin-3-yl ester trichloromethyl ester (246 mg, 0.67 mmol), prepared as described in Example 1(B), in DCM (5 mL) was stirred at room temperature for 2h 15min, under nitrogen atmosphere. The solvent was evaporated under reduced pressure and the crude residue was purified by flash chromatography (silica gel, eluent: petroleum ether/ethyl acetate 7:3) and then by preparative HPLC to afford the title compound (113 mg) as a white gummy solid.
Yield: 50% (white gummy solid); $[\alpha]_D^{20}$ = +20.87° (c=1.33, MeOH); LCMS (RT): 6.29 min (Method D); MS (ES+) gave m/z: 335.3.
$^1$H-NMR (DMSO-$d_6$, 343K), δ (ppm): 7.44 (dd, 2H); 7.20 (dd, 2H); 6.73 (d br, 1H); 4.60 (m, 1H); 3.75 (m, 1H); 3.63 (m, 2H); 3.45 (m, 1H); 3.31 (ddd, 1H); 1.94-1.36 (m, 12H).

Example 8

Cyclohexyl-carbamic acid (S)-1-(4-fluoro-benzoyl)-piperidin-3-yl ester

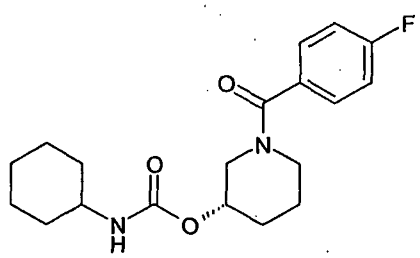

A solution of cyclohexylamine (198 uL, 1.74 mmol) and (S)-carbonic acid 1-(4-fluoro-benzoyl)-piperidin-3-yl ester trichloromethyl ester (320 mg, 0.87 mmol), prepared as described in Example 1(B), in DCM (2 mL) was stirred at room temperature overnight, under nitrogen atmosphere. The solvent was evaporated under reduced pressure and the crude residue was purified by flash chromatography (silica gel, eluent: petroleum ether/ethyl acetate 7:3) and then by preparative HPLC to afford the title compound (87 mg) as a colourless oil.
Yield: 29% (colourless oil); $[\alpha]_D^{20}$ = +19.20° (c=1.08, MeOH); LCMS (RT): 6.54 min (Method D); MS (ES+) gave m/z: 349.3.
$^1$H-NMR (DMSO-$d_6$, 343K), δ (ppm): 7.44 (dd, 2H); 7.20 (dd, 2H); 6.60 (d br, 1H); 4.60 (m, 1H); 3.70-3.54 (m, 2H); 3.44 (dd br, 1H); 3.34 (m, 1H); 1.88 (m, 1H); 1.81-1.63 (m, 7H); 1.55 (m, 2H); 1.34-1.05 (m, 5H).

Example 9

Pyridin-4-yl-carbamic acid (S)-1-(4-fluoro-benzoyl)-piperidin-3-yl ester

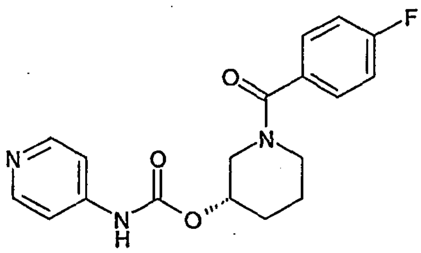

The title compound was obtained following the experimental procedure described in Example 8, starting from (S)-carbonic acid 1-(4-fluoro-benzoyl)-piperidin-3-yl ester trichloromethyl ester, prepared as described in Example 1(B), and 4-amino-pyridine. Pyridin-4-yl-carbamic acid (S)-1-(4-fluoro-benzoyl)-piperidin-3-yl ester (42 mg) was obtained pure after purification by flash chromatography (silica gel, eluent: DCM/MeOH 99:1).
Yield: 14% (off-white solid); LCMS (RT): 5.01 min (Method D); MS (ES+) gave m/z: 344.2.
$^1$H-NMR (DMSO-$d_6$, 343K), δ (ppm): 9.79 (s br, 1H); 8.37 (dd, 2H); 7.45 (dd, 2H); 7.40 (dd, 2H); 7.13 (dd, 2H); 4.79 (m, 1H); 3.76-3.59 (m, 3H); 3.33 (m, 1H); 1.97 (m, 1H); 1.89-1.75 (m, 2H); 1.59 (m, 1H).

Example 10

(4-Fluoro-benzyl)-carbamic acid (S)-1-(4-fluoro-benzoyl)-piperidin-3-yl ester

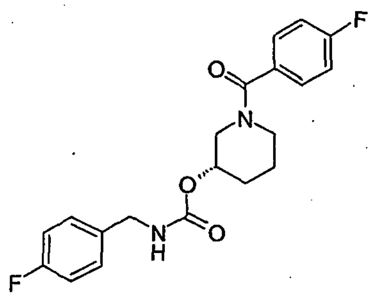

A solution of 4-fluorobenzylamine (62 uL, 0.54 mmol), (S)-carbonic acid 1-(4-fluorobenzoyl)-piperidin-3-yl ester trichloromethyl ester (100 mg, 0.45 mmol), prepared as described in Example 1(B), and triethylamine (125 uL, 0.9 mmol) in DCM (8 mL) was stirred at room temperature overnight, under nitrogen atmosphere. 10% HCl was added and the phases were separated, the organic layer was dried over magnesium sulphate and evaporated to dryness under reduced pressure. The crude residue was purified by flash chromatography (silica gel, eluent gradient: from hexane/ethyl acetate 8:2 to hexane/ethyl acetate 4 :6) and then by preparative HPLC to afford the title compound (55 mg) as a colourless oil. Yield: 33% (colourless oil); $[\alpha]_D^{20}$ = +11.7° (c=0.88, MeOH); LCMS (RT): 6.39 min (Method D); MS (ES+) gave m/z: 375.2. $^1$H-NMR (DMSO-$d_6$, 373K), δ (ppm): 7.42 (dd, 2H); 7.30 (dd, 2H); 7.15 (dd, 2H); 7.11 (m, 1H); 7.07 (dd, 2H); 4.66 (m, IH); 4.18 (d, 2H); 3.64 (dd, 1H); 3.56 (m 1H); 3.48 (dd, 1H); 3.35 (ddd, 1H); 1.90 (m, 1H); 1.82-1.66 (m, 2H); 1.54 (m, 1H).

Example 11

1-[(S)-1-(4-Fluoro-benzoyl)-piperidin-3-yl]-3-(4-fluoro-phenyl)-urea

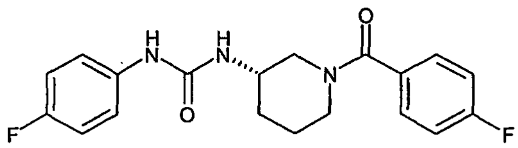

11(A) (S)-3-[3-(4-Fluoro-phenyl)-ureido]-piperidine-1-carboxylic acid tert-butyl ester

4-Fluorophenylisocyanate (137 mg, 1 mmol) was slowly dropped into a solution of (S)-3-amino-1-N-Boc-piperidine (200 mg, 1 mmol) in DCM (2 mL). The reaction mixture was stirred at ambient temperature for 2h and then the solvent was evaporated under reduced pressure to afford a residue oil (337 mg), which was used for the next step without further purification.
Yield: 100%; LCMS (RT): 7.9 min (Method B); MS (ES+) gave m/z: 338.1.

11(B) 1-(4-Fluoro-phenyl)-3-(S)-piperidin-3-yl-ureahydrochloride

4N HCl (dioxane solution, 1 mL) was added to a cooled solution of (S)-3-[3-(4-fluoro-phenyl)-ureido]-piperidine-1-carboxylic acid tert-butyl ester (337 mg, 1mmol) in dioxane (3 mL) at 0°C. The solution was allowed to warm at room temperature and was stirred for 2h. The solvent was evaporated under reduced pressure and the resulting white solid (273 mg) was dried in a vacuum oven at 40°C overnight.
Yield: 100%; LCMS (RT): 4.0 min (Method B); MS (ES+) gave m/z: 238.1.

11(C) 1-[(S)-1-(4-Fluoro-benzoyl)-piperidin-3-yl]-3-(4-fluoro-phenyl)-urea

Triethylamine (400 uL, 3 mmol) was added dropwise at 0°C to a suspension of 1-(4-fluoro-phenyl)-3-(S)-piperidin-3-yl-urea hydrochloride (273 mg, 1.0 mmol) in dichloromethane (10 mL), under nitrogen atmosphere. 4-Fluorobenzoyl chloride (141 uL, 1.2 mmol) was then added while cooling at 0°C and the solution was allowed to warm at room temperature and stirred for 2h. 2N HCl (10 mL) was added and the phases were separated; the organic layer was

washed with 5% $NaHCO_3$ (aq), was dried over sodium sulphate and evaporated under reduced pressure.
The crude residue was purified by flash column chromatography (silica gel, eluent: petroleum ether/ethyl acetate 4:6) to give the title compound (143 mg) as a white solid.
Yield: 40%; mp=228-231 °C; $[\alpha]_D^{20}$ = +40.9° (c=0.5, $CHCl_3$); LCMS (RT): 6.57 min (Method C); MS (ES+) gave m/z: 360.1. $^1$H-NMR (DMSO-$d_6$, 363 K), δ (ppm): 8.14 (s br, 1H); 7.45 (dd, 2H); 7.34 (dd, 2H); 7.14 (dd, 2H); 7.01 (dd, 2H); 6.01 (d br, 1H); 3.79 (dd, 1H); 3.68 (m, 1H); 3.54 (m, 1H); 3.37 (m, 1H); 3.17 (dd, 1H); 1.92 (m, 1H); 1.72 (m, 1H); 1.64-1.48 (m, 2H).

Example 12

(Phenyl)-carbamic acid-1-(4-fluoro-benzoyl)-pyrrolidin-3-yl ester

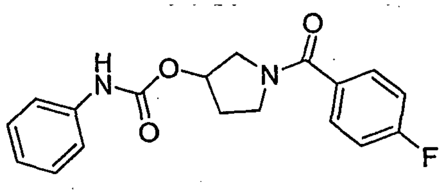

12(A) (4-fluorophenyl)(3-hydroxypyrrolidin-1-yl)methanone

To a solution of pyrrolidin-3-ol (1.7mmol), 150mg, 1 eq) in DCM (20ml) and DIPEA (3.8mmol, 0.64ml, 2.2eq) cooled at -78°C was added 4-fluorobenzoyl chloride (1.7mmol), 0.200ml, leq), the mixture was allowed to warm to RT (just after addition). After 5hour at RT, the reaction indicates the presence of mixture of N- and O-acylated product. The mixture was treated with KOH 3M (10ml) and EtOH (10ml), at 40°C for 12hours. The reaction mixture was concentrated under vacuum to afford an oily residue, which was partitioned between water and EtOAc. The organic layer was successively washed with brine, dried over $MgSO_4$ and concentrated to afford the (4-fluorophenyl)(3-hydroxypyrrolidin-1-yl)methanone as a beige solid (238mg, Yield=66%). LCMS (RT): 1.16 min (Method D); MS (ES+) gave m/z: 210. (4-fluorophenyl)(3-hydroxypyrrolidin-1-yl)methanone was used for the next step without further purification.

12(B) (Phenyl)-carbamic acid-1-(4-fluoro-benzoyl)-pyrrolidin-3-yl ester

A solution of (4-fluorophenyl)(3-hydroxypyrrolidin-1-yl)methanone (0.47mol, 100mg, 1 eq) and Phenylisocyanate (0.47mmole, 0.052ml, 1 eq) in DCM was irradiated under microwaves (2 times at 140°C for 3minutes). The mixture was allowed to stand overnight and the resulting precipitate was filtered and successively washed with a minimal amount of DCM (2 x 0.5ml) and dried to afford the title compound as a white solid (35mg, 23%).
mp= 173-175°C; LCMS (RT): 2.21 min (Method E); MS (ES+) gave m/z: 329.
$^1$H-NMR (DMSO-$d_6$, 300 MHz, 363 K), δ (ppm): 9.75(d, 1H); 7.6(dda, 2H); 7.5(dd, 2H); 7.3(m, 2H); 7.01(dd, 1H); 5.2(d br, 1H); 3.9(m, 1H); 3.6 (m, 2H); 3. 4(dapp, 1H); 2.2(m, 2H).

PHARMACOLOGY:

The compounds provided in the present invention are positive allosteric modulators of mGluR5. As such, these compounds do not activate the mGluR5 by themselves. Instead, the response of mGluR5 to a concentration of glutamate or mGluR5 agonist is increased when compounds of Formula I are present. Compounds of Formula I are expected to have their effect at mGluR5 by virtue of their ability to enhance the function of the receptor.

**EXAMPLE A**

**mGluR5 assay on rat cultured cortical astrocytes**

Under exposure to growth factors (basic fibroblast growth factor, epidermal growth factor), rat cultured astrocytes express group I-Gq coupled mGluR transcripts, namely mGluR5, but none of the splice variants of mGluR1, and as a consequence, a functional expression of mGluR5 receptors (Miller et al. (1995) J. Neurosci. 15:6103-9): The stimulation of mGluR5 receptors with selective agonist CHPG and the full blockade of the glutamate-induced phosphoinositide (PI) hydrolysis and subsequent intracellular calcium mobilization with specific antagonist as MPEP confirm the unique expression of mGluR5 receptors in this preparation.
This preparation was established and used in order to assess the properties of the compounds of the present invention to increase the $Ca^{2+}$ mobilization-induced by glutamate without showing any significant activity when applied in the absence of glutamate.

**Primary cortical astrocytes culture:**

Primary glial cultures were prepared from cortices of Sprague-Dawley 16 to 19 days old embryos using a modification of methods described by Mc Carthy and de Vellis (1980) J. Cell Biol. 85:890-902 and Miller et al. (1995) J. Neurosci. 15(9):6103-9. The cortices were dissected and then dissociated by trituration in a sterile buffer containing 5.36 mM KCl, 0.44 mM $NaHCO_3$, 4.17 mM $KH_2PO_4$, 137 mM NaCl, 0.34 mM $NaH_2PO_4$, 1 g/L glucose. The resulting cell homogenate was plated onto poly-D-lysine precoated T175 flasks (BIOCOAT, Becton Dickinson Biosciences, Erembodegem, Belgium) in Dubelcco's Modified Eagle's Medium (D-MEM GlutaMAX™ I, Invitrogen, Basel, Switzerland) buffered with 25 mM HEPES and 22.7 mM $NaHCO_3$, and supplemented with 4.5g/L glucose, 1 mM pyruvate and 15 % fetal bovine serum (FBS, Invitrogen, Basel, Switzerland), penicillin and streptomycin and incubated at 37°C with 5% $CO_2$. For subsequent seeding, the FBS supplementation was reduced to 10 %. After 12 days, cells were subplated by trypsinisation onto poly-D-lysine precoated 384-well plates at a density of 20.000 cells per well in culture buffer.

**$Ca^{2+}$ mobilization assay using rat cortical astrocytes:**

After 1 day of incubation, cells were washed with Assay buffer containing: 142 mM NaCl, 6 mM KCl, 1 mM $Mg_2SO_4$, 1 mM $CaCl_2$, 20 mM HEPES, 1 g/L glucose, 0.125 mM sulfinpyrazone, pH 7.4. After 60 min of loading with 4 uM Fluo-4 (TefLabs, Austin, TX), the cells were washed three times with 50 μl of PBS Buffer and resuspended in 45 μl of Assay Buffer. The plates were then transferred to a Fluorometric Imaging Plate Reader (FLIPR, Molecular Devices, Sunnyvale, CA) for the assessment of intracellular calcium flux. After monitoring the baseline fluorescence for 10 s, a solution containing 10μM of representative compound of the present invention diluted in Assay Buffer (15 μl of 4X dilutions) was added to the cell plate in the absence or in the presence of 300 nM of glutamate. Under these experimental conditions, this concentration induces less than 20 % of the maximal response of glutamate and was the concentration used to detect the positive allosteric modulator properties of the compounds from the present invention. The final DMSO concentration in the assay was 0.3 %. In each experiment, fluorescence was then monitored as a function of time for 3 minutes and the data analyzed using Microsoft Excel and GraphPad Prism. Each data point was also measured two times.
The results in Figure 1 represent the effect of 10 μM of example #1 on primary cortical mGluR5-expressing cell cultures in the absence or in the presence of 300nM glutamate. Data are expressed as the percentage of maximal response observed with 30μM glutamate applied to the cells. Each bargraph is the mean and S.E.M of duplicate data points and is representative of three independent experiments
The results shown in Example A demonstrate that the compounds described in the present invention do not have an effect per se on mGluR5. Instead, when compounds are added together with an mGluR5 agonist such as glutamate, the effect measured is significantly potentiated compared to the effect of the agonist alone at the same concentration. This data indicates that the compounds of the present invention are positive allosteric modulators of mGluR5 receptors in native preparations.

**EXAMPLE B**

**<u>mGluR5 assay on HEK-expressing rat mGluR5</u>**

**Cell culture**

Positive functional expression of HEK-293 cells stably expressing rat mGluR5 receptor was determined by measuring intracellular $Ca^{2+}$ changes using a Fluorometric Imaging Plate Reader (FLIPR, Molecular Devices, Sunnyvale, CA) in response to glutamate or selective known mGluR5 agonists and antagonists. Rat mGluR5 RT-PCR products in HEK-293 cells were sequenced and found 100% identical to rat mGluR5 Genbank reference sequence (NM_017012). HEK-293 cells expressing rmGluR5 were maintained in media containing DMEM, dialyzed Fetal Bovine Serum (10 %), Glutamax™ (2 mM), Penicillin (100 units/ml), Streptomycin (100 μg/ml), Geneticin (100 μg/ml) and Hygromycin-B (40 μg/ml) at 37°C/5%CO2.

**Fluorescent cell based- Ca2+ mobilization assay**

After one day of incubation, cells were washed with assay buffer containing: 142 mM NaCl, 6 mM KCl, 1 mM

$Mg_2SO_4$, 1 mM $CaCl_2$, 20 mM HEPES, 1 g/L glucose, 0.125 mM sulfinpyrazone, pH 7.4. After 60 min of loading with 4 uM Fluo-4 (TefLabs, Austin, TX), the cells were washed three times with 50 μl of PBS Buffer and resuspended in 45 μl of assay Buffer. The plates were then transferred to a Fluorometric Imaging Plate Reader (FLIPR, Molecular Devices, Sunnyvale, CA) for the assessment of intracellular calcium flux. After monitoring the baseline fluorescence for 10 seconds, increasing concentrations of representative compound (from 0.01 to 60 μM) of the present invention diluted in Assay Buffer (15 μl of 4X dilutions) was added to the cell. The final DMSO concentration in the assay was 0.3 %. In each experiment, fluorescence was then monitored as a function of time for 3 minutes and the data analyzed using Microsoft Excel and GraphPad Prism. Each data point was also measured two times.

Under these experimental conditions, this HEK-rat mGluR5 cell line is able to directly detect positive allosteric modulators without the need of co-addition of glutamate or mGluR5 agonist. Thus, DFB, CPPHA and CDPPB, published reference positive allosteric modulators that are inactive in rat cortical astrocytes culture in the absence of added glutamate (Liu et al (2006) Eur. J. Pharmacol. 536:262-268; Zhang et al (2005); J. Pharmacol. Exp. Ther. 315:1212-1219) are activating, in this system, rat mGluR5 receptors.

The concentration-response curves of representative compounds of the present invention were generated using the Prism GraphPad software (Graph Pad Inc, San Diego, USA). The curves were fitted to a four-parameter logistic equation:

$$(Y=Bottom + (Top-Bottom)/(1+10^\wedge((LogEC50-X)*Hill\ Slope)$$

allowing determination of $EC_{50}$ values.

The Table 1 below represents the mean $EC_{50}$ obtained from at least three independent experiments of selected molecules performed in duplicate.

**Table 1:**

| EXAMPLE | $Ca^{2+}$ Flux* |
|---|---|
| 1 | ++ |
| 2 | ++ |
| 3 | ++ |
| 4 | +++ |
| 5 | +++ |
| 6 | +++ |
| 7 | + |
| 8 | ++ |
| 9 | ++ |
| 10 | ++ |
| 11 | ++ |
| 12 | ++ |
| *Table legend:<br>+: $EC_{50}$ > 10 μM<br>++: 1 μMol <$EC_{50}$ <10 μM<br>+++: $EC_{50}$ <1 μM | |

**EXAMPLE C**

**mGluR5 binding assay**

Activity of compounds of the invention was examined following a radioligand binding technique using whole rat brain and tritiated 2-methyl-6-(phenylethynyl)-pyridine ([$^3$H]-MPEP) as a ligand following similar methods than those described in Gasparini F. et al. (2002) Bioorg. Med. Chem. Lett., 12, 407-409 and in Anderson J.F. et al. J. Pharmacol.

Exp. Ther. 2002, 303, 3, 1044-1051.

**Membrane preparation:**

Cortices were dissected out from brains of 200-300g Sprague-Dawley rats (Charles River Laboratories, L'Arbresle, France). Tissues were homogenized in 10 volumes (vol/wt) of ice-cold 50 mM Hepes-NaOH (pH 7.4) using a Polytron disrupter (Kinematica AG, Luzern, Switzerland) and centrifuged for 30 min at 40,000 g. (4°C). The supernatant was discarded and the pellet washed twice by resuspension in 10 volumes 50 mM HEPES-NaOH. Membranes were then collected by centrifugation and washed before final resuspension in 10 volumes of 20 mM HEPES-NaOH, pH 7.4. Protein concentration was determined by the Bradford method (Bio-Rad protein assay, Reinach, Switzerland) with bovine serum albumin as standard.

**[$^3$H]-MPEP binding experiments:**

Membranes were thawed and resuspended in binding buffer containing 20 mM HEPES-NaOH, 3 mM $MgCl_2$, 3 mM $CaCl_2$, 100 mM NaCl, pH 7.4. Competition studies were carried out by incubating for 1h at 4°C: 3 nM [$^3$H]-MPEP (39 Ci/mmol, Tocris, Cookson Ltd, Bristol, U.K.), 50 μg membrane and a concentration range of 0.003 nM- 30 μM of compounds, for a total reaction volume of 300 μl. The non-specific binding was defined using 30 μM MPEP. Reaction was terminated by rapid filtration over glass-fiber filter plates (Unifilter 96-well GF/B filter plates, Perkin-Elmer, Schwerzenbach, Switzerland) using 4 x 400 μl ice cold buffer using cell harvester (Filtermate, Perkin-Elmer, Downers Grove, USA). Radioactivity was determined by liquid scintillation spectrometry using a 96-well plate reader (TopCount, Perkin-Elmer, Downers Grove, USA).

**Data analysis:**

The inhibition curves were generated using the Prism GraphPad program (Graph Pad Software Inc, San Diego, USA). $IC_{50}$ determinations were made from data obtained from 8 point-concentration response curves using a non linear regression analysis. The mean of $IC_{50}$ obtained from at least three independent experiments of selected molecules performed in duplicate were calculated.

The compounds of this application have $IC_{50}$ values in the range of less than 100 μM. Example # 1 has $IC_{50}$ value of less than 30 μM.

The results shown in examples A, B and C demonstrate that the compounds described in the present invention are positive allosteric modulators of rat mGluR5 receptors. These compounds are active in native systems and are able to inhibit the binding of the prototype mGluR5 allosteric modulator ($^3$H)-MPEP known to bind remotely from the glutamate binding site into the transmembrane domains of mGluR5 receptors (Malherbe et al (2003) Mol. Pharmacol. 64 (4):823-32).

Thus, the positive allosteric modulators provided in the present invention are expected to increase the effectiveness of glutamate or mGluR5 agonists at mGluR5 receptor. Therefore, these positive allosteric modulators are expected to be useful for treatment of various neurological and psychiatric disorders associated with glutamate dysfunction described to be treated herein and others that can be treated by such positive allosteric modulators.

The compounds of the present invention are allosteric modulators of mGluR5 receptors, they are useful for the production of medications, especially for the prevention or treatment of central nervous system disorders as well as other disorders modulated by this receptor.

The compounds of the invention can be administered either alone, or in combination with other pharmaceutical agents effective in the treatment of conditions mentioned above.

<u>FORMULATION EXAMPLES</u>

Typical examples of recipes for the formulation of the invention are as follows:

1) Tablets

| | |
|---|---|
| Compound of the example 1 | 5 to 50 mg |
| Di-calcium phosphate | 20 mg |
| Lactose | 30 mg |
| Talcum | 10 mg |

(continued)

| | |
|---|---|
| Magnesium stearate | 5 mg |
| Potato starch | ad 200 mg |

In this example, the compound of the example 1 can be replaced by the same amount of any of the described examples 1 to 12.

2) Suspension:

An aqueous suspension is prepared for oral administration so that each 1 milliliter contains 1 to 5 mg of one of the described example, 50 mg of sodium carboxymethyl cellulose, 1 mg of sodium benzoate, 500 mg of sorbitol and water ad 1 ml.

3) Injectable

A parenteral composition is prepared by stirring 1.5 % by weight of active ingredient of the invention in 10% by volume propylene glycol and water.

4) Ointment

| | |
|---|---|
| Compound of the example 1 | 5 to 1000 mg |
| Stearyl alcohol | 3 g |
| Lanoline | 5 g |
| White petroleum | 15 g |
| Water | ad 100 g |

In this example, the compound 1 can be replaced by the same amount of any of the described examples 1 to 12.

## Claims

**1.** A compound having the formula I-B or I-C:

wherein according to formula I-B

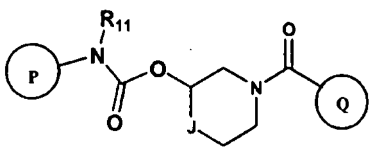

I-B

P is selected from a cycloalkyl, a heterocycloalkyl, an aryl or heteroaryl group of formula

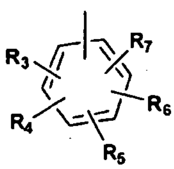 , 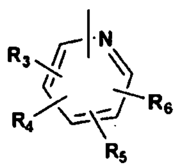 , 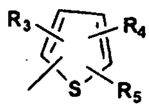 , 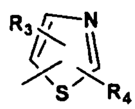 , 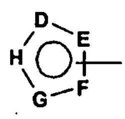

$R_3$, $R_4$, $R_5$, $R_6$, and $R_7$ independently are hydrogen, halogen, -CN, $-NO_2$, $-(C_1-C_6)$alkyl, $-(C_3-C_6)$cycloalkyl,

-($C_3$- $C_7$)cycloalkylalkyl, -($C_2$-$C_6$)alkenyl, -($C_2$-$C_6$)alkynyl, halo-($C_1$-$C_6$)alkyl, heteroaryl, heteroarylalkyl, arylalkyl, aryl, -$OR_8$, -$NR_8R_9$, -C(=$NR_{10}$)$NR_8R_9$, N(=$NR_{10}$)$NR_8R_9$, -$NR_8COR_9$, $NR_8CO_2R_9$, $NR_8SO_2R_9$, -$NR_{10}$CO $NR_8R_9$, -$SR_8$, -S(=O)$R_8$, -S(=O)$_2R_8$, -S(=O)$_2NR_8R_9$, -C(=O)$R_8$, -$COOR_8$, -C(=O)$NR_8R_9$, -C (=$NR_8$)$R_9$, or C(=$NOR_8$)$R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, -($C_1$-$C_6$)alkyl, -O-($C_0$-$C_6$)alkyl, -O-($C_3$- $C_7$)cycloalkylalkyl, -O(aryl), -O(heteroaryl), -O-(-$C_1$- $C_3$)alkylaryl, -O-($C_1$-$C_3$)alkylheteroaryl, -N((-$C_0$- $C_6$)alkyl)(($C_0$-$C_3$)alkylaryl) or -N(($C_0$-$C_6$)alkyl)(($C_0$- $C_3$-)alkylheteroaryl) groups;

$R_8$, $R_9$, $R_{10}$ each independently is hydrogen, ($C_1$-$C_6$)alkyl, ($C_3$- $C_6$)cycloalkyl, ($C_3$-$C_7$)cycloalkylalkyl, ($C_2$-$C_6$) alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$)alkyl, -O-($C_3$-$C_7$)cycloalkylalkyl, -O(aryl), -O(heteroaryl), -N($C_0$- $C_6$-alkyl)$_2$,-N(($C_0$-$C_6$)alkyl)(($C_3$-$C_7$-)cycloalkyl) or -N(($C_0$-$C_6$)alkyl)(aryl) substituents;

D, E, F, G and H represent independently -C($R_3$)=, -C($R_3$)=C($R_4$)-, -C(=O)-,-C(=S)-, -O-, -N=, -N($R_3$)- or -S-;

Q is

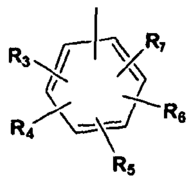

$R_{11}$ represents hydrogen, ($C_1$-$C_6$)alkyl, ($C_3$-$C_6$)cycloalkyl, ($C_3$-$C_7$)cycloalkylalkyl, ($C_2$-$C_6$)alkenyl, ($C_2$- $C_6$) alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$)alkyl, -O-($C_3$- $C_7$)cycloalkylalkyl, -O(aryl), -O(heteroaryl), -N($C_0$- $C_6$-alkyl)$_2$,-N(($C_0$-$C_6$)alkyl)(($C_3$-$C_7$-)cycloalkyl) or -N(($C_0$-$C_6$) alkyl)(aryl) substituents;

J or pharmaceutically compounds; or wherein according to represents a bond, or -$CH_2$; Any N may be an N-oxide; acceptable salts, hydrates or solvates of such formula I-C

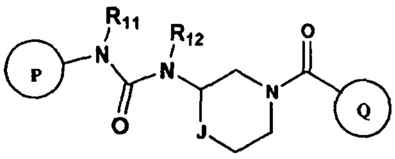

I-C

P is selected from a cycloalkyl, a heterocycloalkyl, an aryl or heteroaryl group of formula

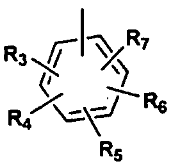

,

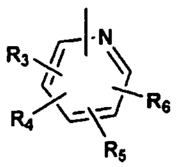

,

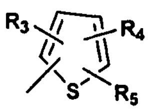

,

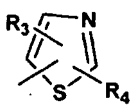

,

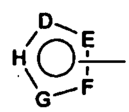

$R_3$, $R_4$, $R_5$, $R_6$, and $R_7$ independently are hydrogen, halogen, -CN, -$NO_2$, -($C_1$-$C_6$)alkyl, -($C_3$-$C_6$)cycloalkyl, -($C_3$- $C_7$)cycloalkylalkyl, -($C_2$-$C_6$)alkenyl, -($C_2$-$C_6$)alkynyl, halo-($C_1$-$C_6$)alkyl, heteroaryl, heteroarylalkyl, arylalkyl, aryl, -$OR_8$, -$NR_8R_9$, -C(=$NR_{10}$)$NR_8R_9$, N(=$NR_{10}$)$NR_8R_9$, -$NR_8COR_9$, $NR_8CO_2R_9$, $NR_8SO_2R_9$, -$NR_{10}$CO $NR_8R_9$, -$SR_8$, -S(=O)$R_8$, -S(=O)$_2R_8$, -S(=O)$_2NR_8R_9$, -C(=O)$R_8$, -$COOR_8$, -C(=O)$NR_8R_9$, -C (=$NR_8$)$R_9$, or C(=$NOR_8$)$R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, -($C_1$-$C_6$)alkyl, -O-($C_0$-$C_6$)alkyl, -O-($C_3$- $C_7$)cycloalkylalkyl,

-O(aryl), -O(heteroaryl), -O-(-$C_1$- $C_3$)alkylaryl, -O-($C_1$-$C_3$)alkylheteroaryl, -N((-$C_0$- $C_6$)alkyl)(($C_0$-$C_3$)alkylaryl) or -N(($C_0$-$C_6$)alkyl)(($C_0$- $C_3$-)alkylheteroaryl) groups;

$R_8$, $R_9$, $R_{10}$ each independently is hydrogen, ($C_1$-$C_6$)alkyl, ($C_3$- $C_6$)cycloalkyl, ($C_3$-$C_7$)cycloalkylalkyl, ($C_2$-$C_6$) alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$)alkyl, -O-($C_3$-$C_7$)cycloalkylalkyl, -O(aryl), -O(heteroaryl), -N($C_0$- $C_6$-alkyl$)_2$,-N(($C_0$-$C_6$)alkyl)(($C_3$-$C_7$-)cyaoalkyl) or -N(($C_0$-$C_6$)alkyl)(aryl) substituents;

D, E, F, G and H represent independently -C($R_3$)=, -C($R_3$)=C($R_4$)-, -C(=O)-,-C(=S)-, -0-, -N=, -N($R_3$)- or -S-;

Q is

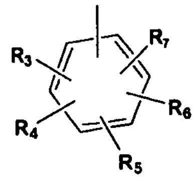

$R_{11}$ and $R_{12}$ each independently is hydrogen, ($C_1$-$C_6$)alkyl, ($C_3$- $C_6$)cycloalkyl, ($C_3$-$C_7$)cycloalkylalkyl, ($C_2$-$C_6$)alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$) alkyl, -O-($C_3$- $C_7$)cycloalkylalkyl, -O(aryl), -O(heteroaryl), -N($C_0$- $C_6$-alkyl$)_2$,-N(($C_0$-$C_6$)alkyl)(($C_3$-$C_7$-)cycloalkyl) or -N(($C_0$-$C_6$)alkyl)(aryl) substituents;

J represents a bond, or -$CH_2$; Any N may be an N-oxide;

or pharmaceutically acceptable salts, hydrates or solvates of such compounds;

with the proviso that: according to formula I-C when $R_{11}$ and $R_{12}$ are both H, and when Q is di-substituted with both 2-OH and 3-$NH_2$, and when J is -$CH_2$, then P cannot be an unsubstituted cyclopentyl, phenyl or benzyl group.

**2.** A compound according to claim 1, wherein

P is

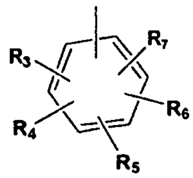

**3.** A compound according to claims 1 or 2, which can exist as optical isomers, wherein said compound is either the racemic mixture or an individual optical isomer.

**4.** A compound according to claim 1, wherein said compound is selected from:

1-[(S)-1-(4-Fluoro-benzoyl)-piperidin-3-yl]-3-(4-fluoro-phenyl)-urea
(4-Fluoro-phenyl)-carbamic acid (S)-1-(4-fluoro-benzoyl)-piperidin-3-yl ester
(4-Chloro-phenyl)-carbamic acid (S)-1-(4-fluoro-benzoyl)-piperidin-3-yl ester
(4-Methoxy-phenyl)-carbamic acid (S)-1-(4-ftuoro-benzoyl)-piperidin-3-yl ester
Phenyl-carbamic acid (S)-1-(4-fluoro-benzoyl)-piperidin-3-yl ester
(3-Fluoro-phenyl)-carbamic acid (S)-1-(4-fluoro-benzoyl)-piperidin-3-yl ester
(2-Fluoro-phenyl)-carbamic acid (S)-1-(4-fluoro-benzoyl)-piperidin-3-yl ester
Cyclopentyl-carbamic acid (S)-1-(4-fluoro-benzoyl)-piperidin-3-yl ester
Cyclohexyl-carbamic acid (S)-1-(4-fluoro-benzoyl)-piperidin-3-yl ester
Pyridin-4-yl-carbamic acid (S)-1-(4-fluoro-benzoyl)-piperidin-3-yl ester
(4-Fluoro-benzyl)-carbamic acid (8)-1-(4-fluoro-benzoyl)-piperidin-3-yl ester
(Phenyl)-carbamic acid-1-(4-fluoro-benzoyl)-pyrrolidin-3-yl ester.

**5.** A pharmaceutical composition comprising a therapeutically effective amount of a compound according to claims 1 to 4 and a pharmaceutically acceptable carrier and/or excipient.

**6.** A compound/composition according to claims 1 to 5, for treating or preventing a condition in a mammal, including a human, the treatment or prevention of which is affected or facilitated by the neuromodulatory effect of mGluR5 allosteric modulators.

**7.** A compound/composition according to claims 1 to 5, for treating or preventing a condition in a mammal, including a human, the treatment or prevention of which is affected or facilitated by the neuromodulatory effect of mGluR5 positive allosteric modulators (enhancer).

**8.** A compound/composition according to claims 1 to 5, for treating or preventing central nervous system disorders selected from the group consisting of anxiety disorders: Agoraphobia, Generalized Anxiety Disorder (GAD), Obsessive-Compulsive Disorder (OCD), Panic Disorder, Posttraumatic Stress Disorder (PTSD), Social Phobia, Other Phobias, Substance-Induced Anxiety Disorder.

**9.** A compound/composition according to claims 1 to 5, for treating or preventing central nervous system disorders selected from the group consisting of childhood disorders: Attention-Deficit/Hyperactivity Disorder).

**10.** A compound/composition according to claims 1 to 5, for treating or preventing central nervous system disorders selected from the group consisting of eating Disorders (Anorexia Nervosa, Bulimia Nervosa).

**11.** A compound/composition according to claims 1 to 5, for treating or preventing central nervous system disorders selected from the group consisting of mood disorders: Bipolar Disorders (I & II), Cyclothymic Disorder, Depression, Dysthymic Disorder, Major Depressive Disorder, Substance-Induced Mood Disorder.

**12.** A compound/composition according to claims 1 to 5, for treating or preventing central nervous system disorders selected from the group consisting of psychotic disorders: Schizophrenia, Delusional Disorder, Schizoaffective Disorder, Schizophreniform Disorder, Substance-Induced Psychotic Disorder.

**13.** A compound/composition according to claims 1 to 5, for treating or preventing central nervous system disorders selected from the group consisting of cognitive disorders: Delirium, Substance-Induced Persisting Delirium, Dementia, Dementia Due to HIV Disease, Dementia Due to Huntington's Disease, Dementia Due to Parkinson's Disease, Dementia of the Alzheimer's Type, Substance-Induced Persisting Dementia, Mild Cognitive Impairment.

**14.** A compound/composition according to claims 1 to 5, for treating or preventing central nervous system disorders selected from the group consisting of personality disorders: Obsessive-Compulsive Personality Disorder, Schizoid, Schizotypal disorder.

**15.** A compound/composition according to claims I to 5, for treating or preventing central nervous system disorders selected from the group consisting of substance-related disorders: Alcohol abuse, Alcohol dependence, Alcohol withdrawal, Alcohol withdrawal delirium, Alcohol- induced psychotic disorder, Amphetamine dependence, Amphetamine withdrawal, Cocaine dependence, Cocaine withdrawal, Nicotine dependence, Nicotine withdrawal, Opioid dependence, Opioid withdrawal.

**16.** A compound/composition according to claims 1 to 5, for treating or preventing inflammatory central nervous system disorders selected from multiple sclerosis form such as benign multiple sclerosis, relapsing-remitting multiple sclerosis, secondary progressive multiple sclerosis, primary progressive multiple sclerosis, progressive-relapsing multiple sclerosis.

**17.** Use of a compound/composition according to claims 1 to 5 in the manufacture of a medicament for a treatment or prevention as defined in any of claims 10 to 18.

**18.** The use of a compound according to claims 1 to 4 to prepare tracers for imaging metabotropic glutamate receptors.

**Patentansprüche**

**1.** Verbindung der Formel I-B oder I-C:

wobei gemäß Formel I-B

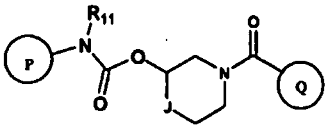

I-B

P ausgewählt ist aus einer Cycloalkyl-, einer Heterocycloalkyl-, einer Aryl- oder Heteroaryl-Gruppe der Formel

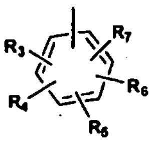 , 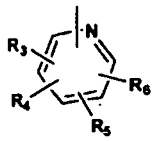 , 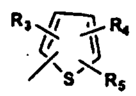 , 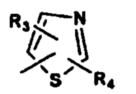 , 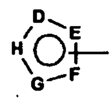

$R_3$, $R_4$, $R_5$, $R_6$ und $R_7$ unabhängig Wasserstoff, Halogen, -CN, $-NO_2$, $-(C_1-C_6)$-Alkyl, $-(C_3-C_6)$-Cycloalkyl, $-(C_3-C_7)$-Cycloalkylalkyl, $-(C_2-C_6)$-Alkenyl, $-(C_2-C_6)$-Alkynyl, Halo-$(C_1-C_6)$-alkyl, Heteroaryl, Heteroarylalkyl, Arylalkyl, Aryl, _$OR_8$, $-NR_8R_9$, $-C(=NR_{10})NR_8R_9$, $-N(=NR_{10})NR_8R_9$, $-NR_8COR_9$, $-NR_8CO_2R_9$, $-NR_8SO_2R_9$, $-NR_{10}CONR_8R_9$, $-SR_8$, $-S(=O)R_8$, $-S(=O)_2R_8$, $-S(=O)_2NR_8R_9$, $-C(=O)R_8$, $-COOR_8$, $-C(=O)NR_8R_9$, $-C(=NR_8)R_9$ oder $-C(=NOR_8)R_9$ Substituenten sind; wobei optional zwei Substituenten mit den dazwischen liegenden Atomen kombiniert sind, um einen bizyklischen Heterocycloalkyl-, Aryl- oder Heteroarylring zu bilden; wobei jeder Ring optional weiter substituiert ist durch 1-5 unabhängige Halogen, -CN, $-(C_1-C_6)$-Alkyl, $-O-(C_0-C_6)$-Alkyl, $-O-(C_3-C_7)$-Cycloalkylalkyl, -O(Aryl), -O(Heteroaryl), $-O-(-C_1-C_3)$-Alkylaryl, $-O-(C_1-C_3)$-Alkylheteroaryl, $-N((-C_0-C_6)$-Alkyl)($(C_0-C_3)$-alkylaryl) oder $-N((C_0-C_6)$-Alkyl)($(C_0-C_3$-)-alkylheteroaryl) Gruppen;
$R_8$, $R_9$, $R_{10}$ jeweils unabhängig Wasserstoff, $(C_1-C_6)$-Alkyl, $(C_3-C_6)$-Cycloalkyl, $(C_3-C_7)$-Cycloalkylalkyl, $(C_2-C_6)$-Alkenyl, $(C_2-C_6)$-Alkynyl, Halo-$(C_1-C_6)$-alkyl, Heterocycloalkyl, Heteroaryl, Heteroarylalkyl, Arylalkyl oder Aryl sind; wobei beliebige davon optional substituiert sind durch 1-5 unabhängige Halogen, -CN, $-(C_1-C_6)$-Alkyl, $-O-(C_0-C_6)$-Alkyl, $-O-(C_3-C_7)$-Cycloalkylalkyl, -O(Aryl), -O(Heteroaryl), $-N(C_0-C_6-Alkyl)_2$, $-N((C_0-C_6$-Alkyl)($(C_3-C_7$-)-cycloalkyl) oder $-N((C_0-C_6)$-Alkyl)(aryl) Substituenten;
D, E, F, G und H unabhängig $-C(R_3)=$, $-C(R_3)=C(R_4)-$, $-C(=O)-$, $-C(=S)-$, -O-, -N=, $-N(R_3)-$ oder -S- repräsentieren;
Q

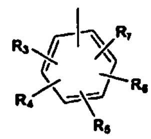

ist;
$R_{11}$ Wasserstoff, $(C_1-C_6)$-Alkyl, $(C_3-C_6)$-Cycloalkyl, $(C_3-C_7)$-Cycloalkylalkyl, $(C_2-C_6)$-Alkenyl, $(C_2-C_6)$-Alkynyl, Halo-$(C_1-C_6)$-alkyl, Heterocycloalkyl, Heteroaryl, Heteroarylalkyl, Arylalkyl oder Aryl repräsentiert; wobei beliebige davon optional substituiert sind durch 1-5 unabhängige Halogen, -CN, $-(C_1-C_6)$-Alkyl, $-O-(C_0-C_6)$-Alkyl, $-O-(C_3-C_7)$-Cycloalkylalkyl, -O(Aryl), -O(Heteroaryl), $-N(Co-C_6-Alkyl)_2$, $-N((C_0-C_6$-Alkyl)($(C_3-C_7$-)-cycloalkyl) oder $-N((Co-C_6)$-Alkyl)(aryl)) Substituenten;
J eine Bindung oder $-CH_2$ repräsentiert;
jedes beliebige N ein N-Oxid sein kann;
oder pharmazeutisch akzeptable Salze, Hydrate oder Solvate solcher Verbindungen; oder
wobei gemäß Formel I-C

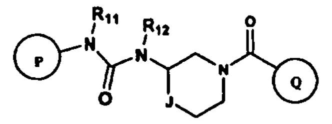

I-C

P ausgewählt ist aus einer Cycloalkyl-, einer Heterocycloalkyl-, einer Aryl- oder Heteroaryl-Gruppe der Formel

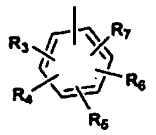 . 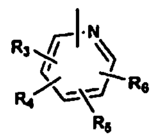 . 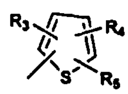 . 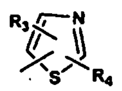 . 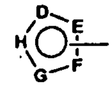

$R_3$, $R_4$, $R_5$, $R_6$ und $R_7$ unabhängig Wasserstoff, Halogen, -CN, -$NO_2$, -($C_1$-$C_6$)-Alkyl, -($C_3$-$C_6$)-Cycloalkyl, -($C_3$-$C_7$)-Cycloalkylalkyl, -($C_2$-$C_6$)-Alkenyl, -($C_2$-$C_6$)-Alkynyl, Halo-($C_1$-$C_6$)-alkyl, Heteroaryl, Heteroarylalkyl, Arylalkyl, Aryl, -$OR_8$, -$NR_8R_9$, -C(=$NR_{10}$)$NR_8R_9$, -N(=$NR_{10}$)$NR_8R_9$, -$NR_8COR_9$, -$NR_8CO_2R_9$, -$NRsSO_2R_9$, -$NR_{10}CONR_8R_9$, -$SR_8$, -S(=O)$R_8$, -S(=)$_2R_8$, -S(=O)$_2NR_8R_9$, -C(=O)$R_8$, -$COOR_8$, -C(=O)$NR_8R_9$, -C(=$NR_8$)$R_9$ oder -C(=$NOR_8$)$R_9$ Substituenten sind; wobei optional zwei Substituenten mit den dazwischen liegenden Atomen kombiniert sind, um einen bizyklischen Heterocycloalkyl-, Aryl- oder Heteroarylring zu bilden; wobei jeder Ring optional weiter substituiert ist durch 1-5 unabhängige Halogen, -CN, -($C_1$-$C_6$)-Alkyl, -O-($C_0$-$C_6$)-Alkyl, -O-($C_3$-$C_7$)-Cycloalkylalkyl, -O(Aryl), -O(Heteroaryl), -O-(-$C_1$-$C_3$)-Alkylaryl, -O-($C_1$-$C_3$)-Alkylheteroaryl, -N((-$C_0$-$C_6$)-Alkyl) (($C_0$-$C_3$)-alkylaryl) oder -N(($C_0$-$C_6$)-Alkyl) (($C_0$-$C_3$-)-alkylheteroaryl) Gruppen;
$R_8$, $R_9$, $R_{10}$ jeweils unabhängig Wasserstoff, ($C_1$-$C_6$)-Alkyl, ($C_3$-$C_6$)-Cycloalkyl, ($C_3$-$C_7$)-Cycloalkylalkyl, ($C_2$-$C_6$)-Alkenyl, ($C_2$-$C_6$)-Alkynyl, Halo-($C_1$-$C_6$)-alkyl, Heterocycloalkyl, Heteroaryl, Heteroarylalkyl, Arylalkyl oder Aryl sind; wobei beliebige davon optional substituiert sind durch 1-5 unabhängige Halogen, -CN, ($C_1$-$C_6$)-Alkyl, -O-($C_0$-$C_6$)-Alkyl, -O-($C_3$-$C_7$)-Cycloalkylalkyl, -O(Aryl), -O(Heteroaryl), -N($C_0$-$C_6$-Alkyl)$_2$, -N(($C_0$-$C_6$-Alkyl)(($C_3$-$C_7$-)-cycloalkyl) oder -N(($C_0$-$C_6$)-Alkyl)(aryl)) Substituenten;
D, E, F, G und H unabhängig -C($R_3$)=, -C($R_3$)=C($R_4$)-, -C(=O)-, -C(=S)-, -O-, -N=, -N($R_3$)- oder -S- repräsentieren;
Q

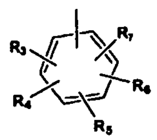

ist;
$R_{11}$ und $R_{12}$ jeweils unabhängig Wasserstoff, ($C_1$-$C_6$)-Alkyl, ($C_3$-$C_6$)-Cycloalkyl, ($C_3$-$C_7$)-Cycloalkylalkyl, ($C_2$-$C_6$)-Alkenyl, ($C_2$-$C_6$)-Alkynyl, Halo-($C_1$-$C_6$)-alkyl, Heterocycloalkyl, Heteroaryl, Heteroarylalkyl, Arylalkyl oder Aryl sind; wobei beliebige davon optional substituiert sind durch 1-5 unabhängige Halogen, -CN, ($C_1$-$C_6$)-Alkyl, -O-($C_0$-$C_6$)-Alkyl, -O-($C_3$-$C_7$)-Cycloalkylalkyl, -O(Aryl), -O(Heteroaryl), -N($C_0$-$C_6$-Alkyl)$_2$, -N(($C_0$-$C_6$-Alkyl)(($C_3$-$C_7$-)-cycloalkyl) oder -N(($C_0$-$C_6$)-Alkyl)(aryl)) Substituenten;
J eine Bindung oder -$CH_2$ repräsentiert;
jedes beliebige N ein N-Oxid sein kann;
oder pharmazeutisch akzeptable Salze, Hydrate oder Solvate solcher Verbindungen;
unter der Voraussetzung, dass: wenn gemäß Formel I-C $R_{11}$ und $R_{12}$ beide H sind und wenn Q disubstituiert ist durch sowohl 2-OH als auch 3-$NH_2$ und wenn J -$CH_2$ ist, P keine unsubstituierte Cyclopentyl-, Phenyl- oder Benzylgruppe sein kann.

**2.** Verbindung nach Anspruch 1, wobei
P

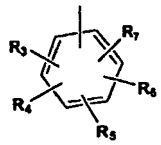

ist.

3. Verbindung nach Anspruch 1 oder 2, die als optische Isomere vorliegen kann, wobei die genannte Verbindung entweder das racemische Gemisch oder ein individuelles optisches Isomer ist.

4. Verbindung nach Anspruch 1, wobei die genannte Verbindung ausgewählt ist aus:

1-[(S)-1-(4-Fluor-benzoyl)-piperidin-3-yl]-3-(4-fluorphenyl)-harnstoff
(4-Fluor-phenyl)-carbaminsäure-(S)-1-(4-fluor-benzoyl)-piperidin-3-yl-ester
(4-Chlor-phenyl)-carbaminsäure-(S)-1-(4-fluor-benzoyl)-piperidin-3-yl-ester
(4-Methoxy-phenyl)-carbaminsäure-(S)-1-(4-fluor-benzoyl)-piperidin-3-yl-ester
Phenyl-carbaminsäure-(S)-1-(4-fluor-benzoyl)-piperidin-3-yl-ester
(3-Fluor-phenyl)-carbaminsäure-(S)-1-(4-fluor-benzoyl)-piperidin-3-yl-ester
(2-Fluor-phenyl)-carbaminsäure-(S)-1-(4-fluor-benzoyl)-piperidin-3-yl-ester
Cyclopentyl-carbaminsäure-(S)-1-(4-fluor-benzoyl)-piperidin-3-yl-ester
Cyclohexyl-carbaminsäure-(S)-1-(4-fluor-benzoyl)-piperidin-3-yl-ester
Pyridin-4-yl-carbaminsäure-(S)-1-(4-fluor-benzoyl)-piperidin-3-yl-ester
(4-Fluor-benzyl)-carbaminsäure-(S)-1-(4-fluor-benzoyl)-piperidin-3-yl-ester
(Phenyl)-carbaminsäure-1-(4-fluor-benzoyl)-pyrrolidin-3-yl-ester.

5. Pharmazeutische Zusammensetzung, die eine therapeutisch wirksame Menge einer Verbindung nach den Ansprüchen 1 bis 4 und einen pharmazeutisch akzeptablen Träger und/oder Exzipienten umfasst.

6. Verbindung/Zusammensetzung nach den Ansprüchen 1 bis 5 zur Behandlung oder Verhütung eines Zustands bei einem Säugetier, einschließlich eines Menschen, dessen Behandlung oder Verhütung durch den neuromodulatorischen Effekt von allosterischen mGluR5-Modulatoren beeinflusst oder erleichtert wird.

7. Verbindung/Zusammensetzung nach den Ansprüchen 1 bis 5 zur Behandlung oder Verhütung eines Zustands bei einem Säugetier, einschließlich eines Menschen, dessen Behandlung oder Verhütung durch den neuromodulatorischen Effekt von positiven allosterischen mGluR5-Modulatoren (Enhancer) beeinflusst oder erleichtert wird.

8. Verbindung/Zusammensetzung nach den Ansprüchen 1 bis 5 zur Behandlung oder Verhütung von Zentralnervensystemstörungen, ausgewählt aus der Gruppe bestehend aus Angststörungen: Agoraphobie, generalisierte Angststörung (GAD), obsessive Zwangsstörung (OCD), Panikstörung, posttraumatische Belastungsstörung (PTSD), soziale Phobie, andere Phobien, substanzinduzierte Angststörung.

9. Verbindung/Zusammensetzung nach den Ansprüchen 1 bis 5 zur Behandlung oder Verhütung von Zentralnervensystemstörungen, ausgewählt aus der Gruppe bestehend aus Kindheitsstörungen: Aufmerksamkeitsdefizit/Hyperaktivitätsstörung.

10. Verbindung/Zusammensetzung nach den Ansprüchen 1 bis 5 zur Behandlung oder Verhütung von Zentralnervensystemstörungen, ausgewählt aus der Gruppe bestehend aus Essstörungen (Magersucht, Ess-Brech-Sucht).

11. Verbindung/Zusammensetzung nach den Ansprüchen 1 bis 5 zur Behandlung oder Verhütung von Zentralnervensystemstörungen, ausgewählt aus der Gruppe bestehend aus Gemütszustandsstörungen: bipolare Störungen (I & II), zyklothyme Störung, Depressionen, dysthyme Störung, majore Depressionsstörung, substanzinduzierte Gemütszustandsstörung.

12. Verbindung/Zusammensetzung nach den Ansprüchen 1 bis 5 zur Behandlung oder Verhütung von Zentralnervensystemstörungen, ausgewählt aus der Gruppe bestehend aus psychotischen Störungen: Schizophrenie, Wahnstörung, schizoaffektive Störung, schizophreniforme Störung, substanzinduzierte psychotische Störung.

**13.** Verbindung/Zusammensetzung nach den Ansprüchen 1 bis 5 zur Behandlung oder Verhütung von Zentralnervensystemstörungen, ausgewählt aus der Gruppe bestehend aus kognitiven Störungen: Delirium, substanzinduziertes anhaltendes Delirium, Demenz, Demenz infolge der HIV-Krankheit, Demenz infolge der Huntington-Krankheit, Demenz infolge der Parkinson-Krankheit, Demenz vom Alzheimer-Typ, substanzinduzierte anhaltende Demenz, leichte kognitive Beeinträchtigung.

**14.** Verbindung/Zusammensetzung nach den Ansprüchen 1 bis 5 zur Behandlung oder Verhütung von Zentralnervensystemstörungen, ausgewählt aus der Gruppe bestehend aus Persönlichkeitsstörungen: Zwangspersönlichkeitsstörung, schizoide, schizotypische Störung.

**15.** Verbindung/Zusammensetzung nach den Ansprüchen 1 bis 5 zur Behandlung oder Verhütung von Zentralnervensystemstörungen, ausgewählt aus der Gruppe bestehend aus substanzbedingten Störungen: Alkoholmissbrauch, Alkoholabhängigkeit, Alkoholentzug, Alkoholentzugsdelirium, Alkohol-induzierte psychotische Störung, Amphetaminabhängigkeit, Amphetaminentzug, Kokainabhängigheit, Kokainentzug, Nikotinabhängigkeit, Nikotinentzug, Opioidabhängigkeit, Opioidentzug.

**16.** Verbindung/Zusammensetzung nach den Ansprüchen 1 bis 5 zur Behandlung oder Verhütung von entzündlichen Zentralnervensystemstörungen, ausgewählt aus Formen der Multiplen Sklerose wie benigne Multiple Sklerose, schubförmig remittierende Multiple Sklerose, sekundärprogressive Multiple Sklerose, primär-progressive Multiple Sklerose, progressiv-schubförmige Multiple Sklerose.

**17.** Verwendung einer Verbindung/Zusammensetzung nach den Ansprüchen 1 bis 5 zur Herstellung eines Medikaments zur Behandlung oder Verhütung wie in einem der Ansprüche 10 bis 18 definiert.

**18.** Verwendung einer Verbindung nach den Ansprüchen 1 bis 4 zur Herstellung von Tracern zur Abbildung von metabotropen Glutamatrezeptoren.

## Revendications

**1.** Composé de formule I-B ou I-C :

où, dans la formule I-B

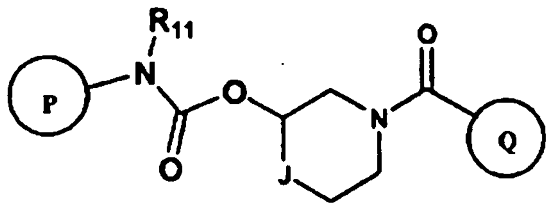

**I-B**

P est sélectionné parmi un groupement cycloalkyle, hétérocycloalkyle, aryle ou hétéroaryle de formule

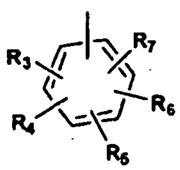 . 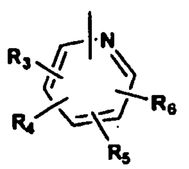 ; 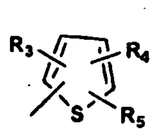 . 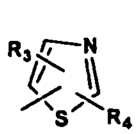 . 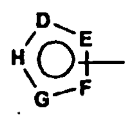 .

$R_3$, $R_4$, $R_5$, $R_6$, et $R_7$ représentent indépendamment un hydrogène, halogène, -CN, -$NO_2$, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en

($C_1$-$C_6$), hétéroaryle, hétéroarylalkyle, arylalkyle, aryle, -$OR_8$, -$NR_8R_9$, -C(=$NR_{10}$)$NR_8R_9$, -N(=$NR_{10}$)$NR_8R_9$, -$NR_8COR_9$, -$NR_8CO_2R_9$, -$NR_8SO_2R_9$, -$NR_{10}$CO $NR_8R_9$, -$SR_8$, -S(=O)$R_8$, -S(=O)$_2R_8$, -S(=O)$_2NR_8R_9$, -C(=O)$R_8$, -$COOR_8$, -C(=O)$NR_8R_9$, -C(=$NR_8$)$R_9$ ou -C(=$NOR_8$)$R_9$; où deux de ces substituants sont en option combinés aux atomes intervenants pour former un noyau hétérocycloalkyle, aryle ou hétéroaryle bicyclique ; chaque noyau étant en option également substitué par 1-5 substituants indépendamment sélectionnés parmi un halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -O-alkylaryle en ($C_1$-$C_3$), -O-alkylhétéroaryle en ($C_1$-$C_3$), -N-[alkyle en ($C_0$-$C_6$)-alkylaryle en ($C_0$-$C_3$)] ou -N-[alkyle en ($C_0$-$C_6$)-alkylhétéroarylaryle en ($C_0$-$C_3$)];

$R_8$, $R_9$ et $R_{10}$ représentent chacun indépendamment un hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant en option substitué par 1-5 substituants indépendamment sélectionnés parmi un halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)-cycloalkyle en ($C_3$-$C_7$)] ou -N-[alkyle en ($C_0$-$C_6$)-aryle] ;

D, E, F, G et H représentent indépendamment un groupement -C($R_3$)=, -C($R_3$)=C($R_4$)-, -C(=O)-, -C(=S)-, -O-, -N=, -N($R_3$)- ou -S- ;

Q représente

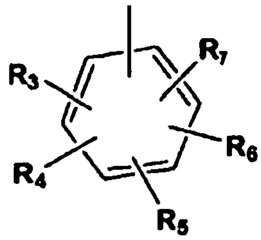

$R_{11}$ représente un hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant en option substitué par 1-5 substituants indépendamment sélectionnés parmi un halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)-Cycloalkyle en ($C_3$-$C_7$)] ou -N-[alkyle en ($C_0$-$C_6$)-aryle] ;

J représente une liaison ou -$CH_2$;

Tout N peut représenter un N-oxyde ;

ou sels, hydrates ou solvates pharmaceutiquement acceptables de tels composés ; ou

où, dans la formule I-C

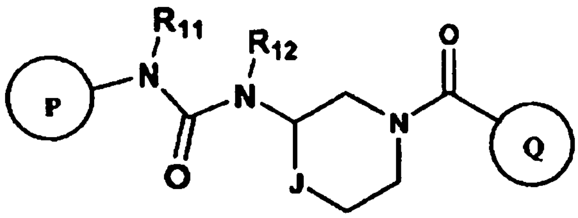

I-C

P est sélectionné parmi un groupement cycloalkyle, hétérocycloalkyle, aryle ou hétéroaryle de formule

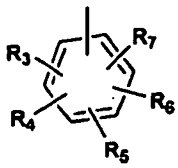 , 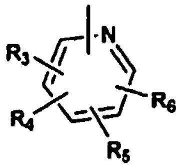 , 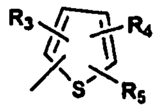 , 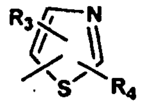 , 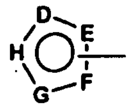

$R_3$, $R_4$, $R_5$, $R_6$, et $R_7$ représentent indépendamment un hydrogène, halogène, -CN, $-NO_2$, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétéroaryle, hétéroarylalkyle, arylalkyle, aryle, $-OR_8$, $-NR_8R_9$, $-C(=NR_{10})NR_8R_9$, $-N(=NR_{10})NR_8R_9$, $-NR_8COR_9$, $-NR_8CO_2R_9$, $-NR_8SO_2R_9$, $-NR_{10}CO\ NR_8R_9$, $-SR_8$, $-S(=O)R_8$, $-S(=O)_2R_8$, $-S(=O)_2NR_8R_9$, $-C(=O)R_8$, $-COOR_8$, $-C(=O)NR_8R_9$, $-C(=NR_8)R_9$ ou $-C(=NOR_8)R_9$ ; où deux de ces substituants sont en option combinés aux atomes intervenants pour former un noyau hétérocycloalkyle, aryle ou hétéroaryle bicyclique ; chaque noyau étant en option également substitué par 1-5 substituants indépendamment sélectionnés parmi un halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -O-alkylaryle en ($C_1$-$C_3$), -O-alkylhétéroaryle en ($C_1$-$C_3$), -N-[alkyle en ($C_0$-$C_6$)-alkylaryle en ($C_0$-$C_3$)] ou -N-[alkyle en ($C_0$-$C_6$)-alkylhétéroarylaryle en ($C_0$-$C_3$)] ;

$R_8$, $R_9$ et $R_{10}$ représentent chacun indépendamment un hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant en option substitué par 1-5 substituants indépendamment sélectionnés parmi un halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)-cycloalkyle en ($C_3$-$C_7$)] ou -N-[alkyle en ($C_0$-$C_6$)-aryle] ;

D, E, F, G et H représentent indépendamment un groupement $-C(R_3)=$, $-C(R_3)=C(R_4)-$,- C(=O)-, -C(=S)-, -O-, -N= $-N(R_3)-$ ou -S- ;

Q représente

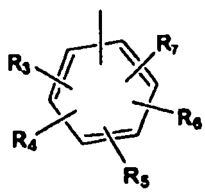

$R_{11}$ et $R_{12}$ représentent chacun indépendamment un hydrogène, alkyle en ($C_1$-$C_6$), cycloalkyle en ($C_3$-$C_6$), cycloalkylalkyle en ($C_3$-$C_7$), alkényle en ($C_2$-$C_6$), alkynyle en ($C_2$-$C_6$), halo-alkyle en ($C_1$-$C_6$), hétérocycloalkyle, hétéroaryle, hétéroarylalkyle, arylalkyle ou aryle ; l'un quelconque de ceux-ci étant en option substitué par 1-5 substituants indépendamment sélectionnés parmi un halogène, -CN, alkyle en ($C_1$-$C_6$), -O-alkyle en ($C_0$-$C_6$), -O-cycloalkylalkyle en ($C_3$-$C_7$), -O-aryle, -O-hétéroaryle, -N-[alkyle en ($C_0$-$C_6$)]$_2$, -N-[alkyle en ($C_0$-$C_6$)-cycloalkyle en ($C_3$-$C_7$)] ou -N-[alkyle en ($C_0$-$C_6$)-aryle] ;

J représente une liaison ou $-CH_2$ ;

Tout N peut représenter un N-oxyde ;

ou sels, hydrates ou solvates pharmaceutiquement acceptables de tels composés ;

à condition que : dans la formule I-C, quand $R_{11}$ et $R_{12}$ représentent tous deux H, Q est disubstitué par 2-OH et $3-NH_2$ et J représente $-CH_2$, P ne peut pas représenter un groupement cyclopentyle, phényle ou benzyle non substitué.

**2.** Composé selon la revendication 1, où P représente

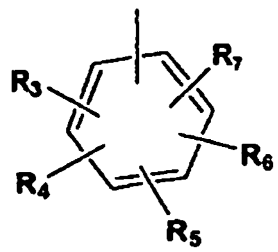

3. Composé selon la revendication 1 ou 2, qui peut exister sous la forme d'isomères optiques, où ledit composé est soit le mélange racémique, soit un isomère optique individuel.

4. Composé selon la revendication 1, où ledit composé est sélectionné parmi les suivants :

1-[(S)-1-(4-Fluoro-benzoyl)-pipéridin-3-yl]-3-(4-fluoro-phényl)-urée
Ester (S)-1-(4-fluoro-benzoyl)-pipéridin-3-ylique de l'acide (4-fluoro-phényl)-carbamique
Ester (S)-1-(4-fluoro-benzoyl)-pipéridin-3-ylique de l'acide (4-chloro-phényl)-carbamique
Ester (S)-1-(4-fluoro-benzoyl)-pipéridin-3-ylique de l'acide (4-méthoxy-phényl)-carbamique
Ester (S)-1-(4-fluoro-benzoyl)-pipéridin-3-ylique de l'acide phényl-carbamique
Ester (S)-1-(4-fluoro-benzoyl)-pipéridin-3-ylique de l'acide (3-fluoro-phényl)-carbamique
Ester (S)-1-(4-fluoro-benzoyl)-pipéridin-3-ylique de l'acide (2-fluoro-phényl)-carbamique
Ester (S)-1-(4-fluoro-benzoyl)-pipéridin-3-ylique de l'acide cyclopentyl-carbamique
Ester (S)-1-(4-fluoro-benzoyl)-pipéridin-3-ylique de l'acide cyclohexyl-carbamique
Ester (S)-1-(4-fluoro-benzoyl)-pipéridin-3-ylique de l'acide pyridin-4-yl-carbamique
Ester (S)-1-(4-fluoro-benzoyl)-pipéridin-3-ylique de l'acide (4-fluoro-benzyl)-carbamique
Ester 1-(4-fluoro-benzoyl)-pyrrolidin-3-ylique de l'acide phényl-carbamique.

5. Composition pharmaceutique comprenant une quantité thérapeutiquement efficace d'un composé selon les revendications 1 à 4 et un véhicule et/ou excipient pharmaceutiquement acceptable.

6. Composé/composition selon les revendications 1 à 5, pour le traitement ou la prévention chez un mammifère, y compris un être humain, d'une maladie dont le traitement ou la prévention est influencé ou facilité par l'effet neuromodulateur de modulateurs allostériques du récepteur mGluR5.

7. Composé/composition selon les revendications 1 à 5, pour le traitement ou la prévention chez un mammifère, y compris un être humain, d'une maladie dont le traitement ou la prévention est influencé ou facilité par l'effet neuromodulateur de modulateurs allostériques positifs (stimulateurs) du récepteur mGluR5.

8. Composé/composition selon les revendications 1 à 5, pour le traitement ou la prévention de maladies du système nerveux central sélectionnées dans le groupe consistant en des troubles anxieux : agoraphobie, trouble anxieux généralisé (TAG), trouble obsessionnel compulsif (TOC), trouble panique, syndrome de stress post-traumatique (SSPT), phobie sociale, autres phobies, trouble anxieux induit par une substance.

9. Composé/composition selon les revendications 1 à 5, pour le traitement ou la prévention de maladies du système nerveux central sélectionnées dans le groupe consistant en des maladies infantiles : trouble de déficit de l'attention et hyperactivité.

10. Composé/composition selon les revendications 1 à 5, pour le traitement ou la prévention de maladies du système nerveux central sélectionnées dans le groupe consistant en des troubles du comportement alimentaire : anorexie mentale, boulimie.

11. Composé/composition selon les revendications 1 à 5, pour le traitement ou la prévention de maladies du système nerveux central sélectionnées dans le groupe consistant en des troubles de l'humeur : maladies à forme bipolaire (I & II), trouble cyclothymique, dépression, trouble dysthymique, trouble dépressif majeur, trouble de l'humeur induit par une substance.

**12.** Composé/composition selon les revendications 1 à 5, pour le traitement ou la prévention de maladies du système nerveux central sélectionnées dans le groupe consistant en des maladies psychotiques : schizophrénie, trouble délirant, trouble schizoaffectif, trouble schizophréniforme, trouble psychotique induit par une substance.

**13.** Composé/composition selon les revendications 1 à 5, pour le traitement ou la prévention de maladies du système nerveux central sélectionnées dans le groupe consistant en des troubles cognitifs : délire, délire persistant induit par une substance, démence, complexe démentiel associé au VIH, démence de la maladie de Huntington, démence de la maladie de Parkinson, démence de type Alzheimer, démence persistante induite par une substance, déclin cognitif léger.

**14.** Composé/composition selon les revendications 1 à 5, pour le traitement ou la prévention de maladies du système nerveux central sélectionnées dans le groupe consistant en des troubles de la personnalité : trouble de la personnalité obsessionnelle-compulsive, schizoïdie, trouble schizotypique.

**15.** Composé/composition selon les revendications 1 à 5, pour le traitement ou la prévention de maladies du système nerveux central sélectionnées dans le groupe consistant en des troubles liés à l'usage d'une substance : usage abusif d'alcool, alcoolodépendance, sevrage éthylique, délire du syndrome de sevrage éthylique, trouble psychotique d'origine éthylique, dépendance aux amphétamines, sevrage des amphétamines, dépendance à la cocaïne, sevrage de la cocaïne, dépendance à la nicotine, sevrage de la nicotine, dépendance aux opioïdes, sevrage des opioïdes.

**16.** Composé/composition selon les revendications 1 à 5, pour le traitement ou la prévention de maladies inflammatoires du système nerveux central sélectionnées dans le groupe consistant en une forme de sclérose en plaques comme la forme bénigne de sclérose en plaques, la forme récurrente-rémittente de sclérose en plaques, la forme progressive secondaire de sclérose en plaques, la forme progressive primaire de sclérose en plaques et la forme progressive récurrente de sclérose en plaques.

**17.** Utilisation d'un composé/d'une composition selon les revendications 1 à 5 dans la fabrication d'un médicament pour le traitement ou la prévention d'une maladie telle que définie à l'une quelconque des revendications 10 à 18.

**18.** Utilisation d'un composé selon les revendications 1 à 4 pour la préparation de traceurs pour l'imagerie des récepteurs métabotropes au glutamate.

**Figure 1**

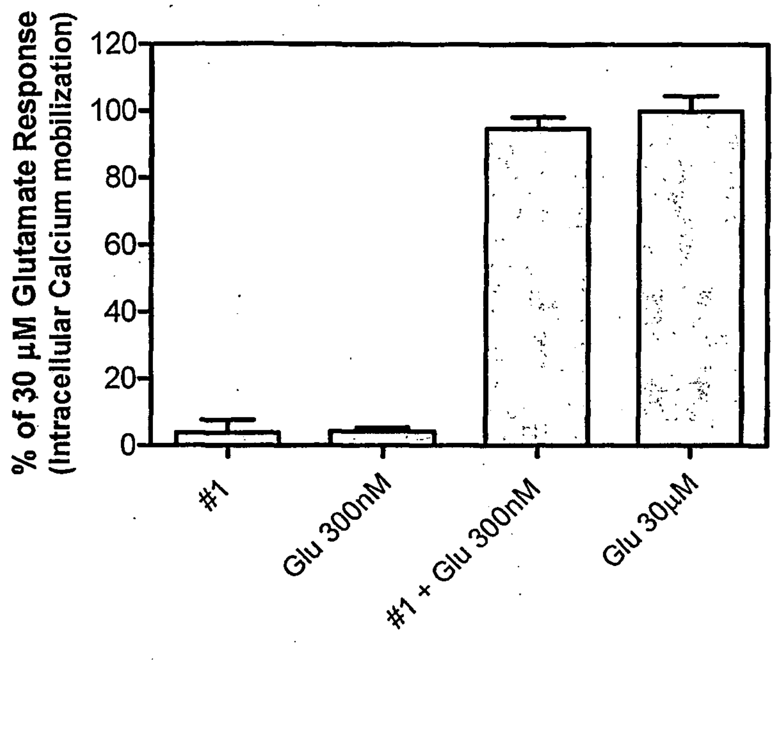
% of 30 µM Glutamate Response
(Intracellular Calcium mobilization)
120
100
80
60
40
20
0
#1
Glu 300nM
#1 + Glu 300nM
Glu 30µM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004087048 A
- WO 2005087048 A
- WO 2005044797 A
- WO 2004014370 A
- WO 2004033440 A


### Non-patent literature cited in the description


- **Nakanishi S et al.** *Brain Res. Rev.,* 1998, vol. 26, 230-235
- **Schoepp DD et al.** *Neuropharmacology,* 1999, vol. 38, 1431-1476
- **Lujan R et al.** *Eur. J. Neurosci.,* 1996, vol. 8, 1488-500
- **Lujan R et al.** *J. Chem. Neuroanat.,* 1997, vol. 13, 219-41
- **Romano C et al.** *J. Comp. Neurol.,* 1995, vol. 355, 455-69
- **Brauner-Osbome H et al.** *J. Med. Chem.,* 2000, vol. 43, 2609-45
- **Bordi F ; Ugolini A.** *Prog. Neurobiol.,* 1999, vol. 59, 55-79
- **Spooren W et al.** *Behav. Pharmacol.,* 2003, vol. 14, 257-77
- **Goff DC ; Coyle JT.** *Am. J. Psychiatry,* 2001, vol. 158, 1367-1377
- **Carlsson A et al.** *Annu. Rev. Pharmacol. Toxicol.,* 2001, vol. 41, 237-260
- **Devon RS et al.** *Mol. Psychiatry.,* 2001, vol. 6, 311-4
- **Ohnuma T et al.** *Brain Res. Mol. Brain Res.,* 1998, vol. 56, 207-17
- **Mannaioni G et al.** *Neurosci.,* 2001, vol. 21, 5925-34
- **Awad H et al.** *J. Neurosci.,* 2000, vol. 20, 7871-7879
- **Pisani A et al.** *Neuroscience,* 2001, vol. 106, 579-87
- **Benquet P et al.** *J. Neurosci.,* 2002, vol. 22, 9679-86
- **Martin SJ et al.** *Annu. Rev. Neurosci.,* 2000, vol. 23, 649-711
- **Baudry M ; Lynch G.** *Neurobiol. Learn. Mem.,* 2001, vol. 76, 284-297
- **Lu et al.** *J. Neurosci.,* 1997, vol. 17, 5196-5205
- **Schulz B et al.** *Neuropharmacology,* 2001, vol. 41, 1-7
- **Jia Z et al.** *Physiol. Behav.,* 2001, vol. 73, 793-802
- **Rodrigues et al.** *J. Neurosci.,* 2002, vol. 22, 5219-5229
- **Kinney GG et al.** *J. Pharmacol. Exp. Ther.,* 2003, vol. 306 (1), 116-123
- **Knoflach F et al.** *Proc. Natl. Acad. Sci. U S A.,* 2001, vol. 98, 13402-13407
- **O'Brien JA et al.** *Mol. Pharmacol.,* 2003, vol. 64, 731-40
- **Johnson K et al.** *Neuropharmacology,* 2002, vol. 43, 291
- **Johnson MP et al.** *J. Med. Chem.,* 2003, vol. 46, 3189-92
- **Marino MJ et al.** *Proc. Natl. Acad. Sci. U S A.,* 2003, vol. 100 (23), 13668-73
- **Mutel V.** *Expert Opin. Ther. Patents,* 2002, vol. 12, 1-8
- **Kew JN.** *Pharmacol. Ther.,* 2004, vol. 104 (3), 233-44
- **Johnson MP et al.** *Biochem. Soc. Trans.,* 2004, vol. 32, 881-7
- **O'Brien JA.** *J. Pharmacol. Exp. Ther.,* 2004, vol. 309, 568-77
- **Lindsley et al.** *J. Med. Chem.,* 2004, vol. 47, 5825-8
- **Kinney GG et al.** *J. Pharmacol. Exp. Ther.,* 2005, vol. 313, 199-206
- **Green T.W. ; Wuts P.G.M.** Protecting Groups in Organic Synthesis. John Wiley et Sons, 1991
- **Eliel E.L. ; Wilen S.H. ; Mander L.N.** Stereochemishy of Organic Compounds. Wiley-Interscience, 1984
- **Katrizky A.R. ; Rees C.W.** Comprehensive Heterocyclic Chemistry. Pergamon Press, 1984
- *Tetrahedron Lett.,* 1992, 3583-3586
- *Bioorg. Med Chem,* 2003, 4315
- *J. Org. Chem.,* 1981, vol. 46, 3519-3521
- *Tetrahedron,* 1998, 6757
- **Smith M.B. ; March J.** March's advanced Organic Chemistry. John Wiley &Sons, 2001
- **French ; Wirtel.** *Am. Chem. J,* 1926, vol. 48, 1736

- **Stahl P.H. ; Wermuth C.G.** Handbook of Pharmaceuticals Salts, Properties, Selection and Use. Wiley, 2002
- **Miller et al.** *J. Neurosci.,* 1995, vol. 15, 6103-9
- **Mc Carthy ; de Vellis.** *J. Cell Biol.,* 1980, vol. 85, 890-902
- **Miller et al.** *J. Neurosci.,* 1995, vol. 15 (9), 6103-9
- **Liu et al.** *Eur. J. Pharmacol.,* 2006, vol. 536, 262-268
- **Zhang et al.** *J. Pharmacol. Exp. Ther.,* 2005, vol. 315, 1212-1219
- **Gasparini F. et al.** *Bioorg. Med. Chem. Lett.,* 2002, vol. 12, 407-409
- **Anderson J.F. et al.** *J. Pharmacol. Exp. Ther.,* 2002, vol. 303 (3), 1044-1051
- **Malherbe et al.** *Mol. Pharmacol.,* 2003, vol. 64 (4), 823-32